# EUROPEAN PATENT SPECIFICATION

(11) **EP 1 272 669 B1**
(45) Date of publication and mention of the grant of the patent: **10.06.2009**
(21) Application number: 01927056.0
(22) Date of filing: 13.04.2001
(51) Int. Cl.: C12N 15/10, C12N 15/63, C12N 15/67, C12N 15/80, C12Q 1/02, C12Q 1/25, C12Q 1/68, C12N 1/15

(54) **HIGH-THROUGHPUT SCREENING OF EXPRESSED DNA LIBRARIES IN FILAMENTOUS FUNGI**
DURCHMUSTERUNG VON BIBLIOTHEKEN EXPRIMIERTER DNA IN FILAMENTÖSEN PILZEN MIT HOHER DURCHSATZRATE
CRIBLAGE A DEBIT ELEVE DE BIBLIOTHEQUES D'ADN EXPRIMEES DANS DES CHAMPIGNONS FILAMENTEUX

(30) Priority: 13.04.2000 WO PCT/US00/10199
(43) Date of publication of application: 08.01.2003
(73) Proprietor: Dyadic International (USA), Inc., Jupiter, FL 33477 (US)
(72) Inventor: PUNT, Peter, Jan, NL-3994 XT Houten (NL); VAN ZEIJL, Cornelia, NL-3451 EP Vleuten-de Meern (NL); VAN DEN HONDEL, Cornelius, NL-2804 PZ Gouda (NL); EMALFARB, Mark Aaron, Jupiter, FL (US)
(74) Representative: van Westenbrugge, Andries
(86) International application number: PCT/US2001/012335
(87) International publication number: WO 2001/079558

(56) References cited:
- WO-A-93/11249
- WO-A-97/26330
- WO-A-99/32617
- US-A- 4 816 405
- US-A- 5 536 661
- US-A- 5 578 463
- US-A- 5 695 985
- BERGES T ET AL: "CLONING OF AN ASPERGILLUS NIGER INVERTASE GENE BY EXPRESSION IN TRICHODERMA REESEI" CURRENT GENETICS, NEW YORK, NY, US, vol. 24, 1993, pages 53-59, XP002948214 ISSN: 0172-8083

## Description

### SUMMARY OF THE INVENTION

The invention provides a method for the expression and subsequent screening of DNA libraries, particularly synthetic, genomic, and cDNA libraries, in filamentous fungal hosts. The system employs transformed or transfected filamentous fungal strains which generate transferable reproductive elements, for example by efficient sporulation, in submerged culture. The fungi preferably exhibit a morphology that minimizes or eliminates the formation of entangled mycelia. Particularly preferred fungal strains are also capable of expressing isolatable quantities of exogenous proteins for evaluation. The mutant fungal strains of the invention are particularly well-suited for high-throughput screening techniques, due to their production of transferable reproductive elements, high levels of expression, and very low culture viscosity.

### BACKGROUND OF THE INVENTION.

Naturally-occurring populations of microorganisms exhibit a wide array of biochemical and metabolic diversity. Due in part to difficulties in isolating and culturing many microorganisms, a vast number of potentially valuable proteins and polypeptides present in these populations have escaped identification. Indeed, it has been estimated that less than one percent of the world's microorganisms have been cultured to date. There remains a pressing need for new approaches to the characterization of proteins, polypeptides and metabolites from as-yet uncultivated, unidentified microorganisms, and also from known microorganisms. (The term "protein" as used hereinafter should be understood to encompass peptides and polypeptides as well.) There also remains a need for new approaches to the identification and isolation of the genes encoding these proteins, so as to enable the modification and/or production of the proteins.

One approach to this problem has been described by Short in U.S. Patents 5,958,672; 6,001,574, 6,030,779, and 6,057,103. In this approach, a genomic DNA library is prepared directly from an environmental sample (*e*.*g*. a soil sample), with or without making an attempt to isolate or culture any organisms that might be present. The DNA library is expressed in *E. coli,* and the expressed proteins are screened for a property or activity of interest. Short alludes to, but does not describe or enable, the use of fungal host cells in this method.

The approach as described suffers from several serious disadvantages, one of which is that *E. coli* does not effectively express genes having introns. Roughly 90% of the species of microorganisms in soil are eukaryotes (principally fungi), which generally do have introns in their genomic DNA. Given that there are already about 100,000 species of eumycotan fungi known, with an estimated 1,000,000 yet to be discovered (B.Kendrick, The Fifth Kingdom, Mycologue Publications 1999), the potential for protein and metabolite diversity is far higher among the fungal genomes, but the presence of introns puts most of the fungal protein and metabolite repertoire out of the reach of bacterial expression systems. Not only are many classes of enzymes (*e*.*g*., secretory fungal lignin peroxidases and manganese-dependent peroxidases) unique to fungi, but there are many fungal proteins, including enzymes (*e*.*g*. lignin peroxidases, *A. niger* invertase), that are glycosylated, and such proteins would not be glycosylated if expressed by *E. coli.* The much higher number and greater size and complexity of fungal genomes, the uniqueness of many fungal proteins, and the glycosylation of many fungal proteins, all indicate that the fraction of microbial protein and metabolite diversity in a given environmental sample that could be actually detected by bacterial expression of genomic DNA is considerably less than 10%.

Due in part to the spread of AIDS and the rising population of organ transplant recipients, there is a growing population of immune-compromised or immuno-supressed individuals, and the number and variety of fungal infections has grown apace (*Infect. Med.* **16**:380-382, 385-386 (1999)). There is a need to identify and characterize proteins from pathogenic fungi in the ongoing search for new targets for anti-fungal drugs, which requires the capability to screen DNA libraries derived from fungal genomes. Again, the presence of introns in fungal genomes makes expression of genomic DNA libraries difficult in most currently available bacterial hosts. There has also been a rise in the prevalence of antibiotic-resistant bacterial infections, creating a need for high-throughput screening for new fungal metabolites having antibiotic activity.

Eukaryotic genomes of higher organisms are also too complex for comprehensive expression of DNA libraries in bacteria. When all eukaryotic species are considered, bacteria represent only about 0.3% of all known species (E.O. Wilson, "The Current State of Biological Diversity", in Biodiversity, National Academy Press, Washington DC, 1988, Chapter 1); thus the fraction of the world's genetic diversity accessible to bacterial expression systems is extremely limited.

To avoid problems with introns, it is possible to prepare a cDNA library and express it in bacteria. However, this approach relies upon the presence of RNA transcripts, and any genes not actively being transcribed will not be represented in the library. Many desirable proteins are expressed only under specific conditions (*e*.*g*., virulence factors in pathogenic fungi) and these conditions may not exist at the time the mRNA is harvested. Furthermore, in order to obtain sufficient RNA to prepare a cDNA library, it is necessary to culture a fair amount of the organism. For organisms in environmental samples that do not grow well in culture, or novel microorganisms for which appropriate culture conditions are unknown, sufficient RNA will not be readily or reliably obtained. In contrast, sufficient genomic DNA can be obtained from a very small number of individual cells by PCR amplification, using either random primers or primers designed to favor certain classes of genes. Finally, genes that are highly expressed in an organism will tend to be over-represented in the mRNA, and thus over-represented at the expense of minimally-expressed genes in a cDNA library. In order to have a high level of coverage of the mRNA species present, a much larger number of clones must be screened if a cDNA library is employed instead of a genomic library, since the latter will have a more nearly equal representation of the variety of genes present. Clearly it is more desirable to screen a genomic DNA library if at all possible.

Also, *E. coli* is incapable of secretion of many proteins, and thus is undesirable as a host cell for screening purposes where the screening relies upon secretion of the gene product. An additional disadvantage for *E. coli,* and for bacterial hosts in general, is that prokaryotes cannot provide many of the post-translational modifications required for the activity of numerous eukaryotic proteins. In addition to glycosylation, subunit cleavage, disulfide bond formation, and proper folding of proteins are examples of the post-translational processing often required to produce an active protein.

To ensure such processing one can sometimes use mammalian cells, but mammalian cells are difficult to maintain, require expensive media, and are not generally transformed with high efficiency. Such transformation systems are therefore not convenient for high-throughput screening of proteins, although efforts have been made to employ mammalian cells as hosts for cDNA library screening (Schouten et al., WO 99/64582). An approach involving fusion of transformed protoplasts with mammalian cells prior to library screening has been described (U.S. patent 5,989,814), but expression of the protein library occurs in bacteria or yeast prior to cell fusion. There have been efforts to modify glycosylation patterns enzymatically after expression in host cells (Meynial-Salles and Combes, J. Biotechnol., 46:1-14 (1996)), but such methods must be tailored for specific products and are not suitable for expression of proteins from a DNA library. More recently, Maras et al., Eur. J. Biochem., 249:701-707 (1997) (see also US patent 5,834,251) have described a strain of *Tichoderma reesei* engineered to express human GlcNAc transferase 1. The enzyme transfers N-acetylglucosamine to mannose residues on other expressed exogenous proteins, a first step toward more closely approximating natural mammalian products.

The use of yeast as host cells solves some of the above problems, but introduces others. Yeast tend to hyper-glycosylate exogenous proteins (Bretthauer and Castellino, 1999, Biotechnol. Appl.. Biochem. 30:193-200), and the altered glycosylation patterns often render expressed mammalian proteins highly antigenic (C. Ballou, in Molecular Biology of the Yeast Sacccharomyces, J. Strathern et al., eds., Cold Spring Harbor Laboratory Press, NY, 1982, 335-360). Although yeast are capable of coping with a limited number of introns, they are not generally capable of handling complex genes from higher species such as vertebrates. Even genes from filamentous fungi are usually too complex for yeast to transcribe efficiently, and this problem is compounded by differences in expression and splicing sequences between yeast and filamentous fungi (see *e*.*g*., M. Innis et al., Science 1985 228:21-26). Despite these drawbacks, transformation and expression systems for yeast have been extensively developed, generally for use with cDNA libraries. Yeast expression systems have been developed which are used to screen for naturally secreted and membrane proteins of mammalian origin (Klein, et al., Proc. Natl. Acad. Sci. USA 1996 93:7108-7113; Treco, U.S. patent 5,783,385), and for heterologous fungal proteins (Dalboge and Heldt-Hansen, Mol. Gen. Genet. 243:253-260 (1994)) and mammalian proteins (Tekamp-Olson and Meryweather, U.S. patent 6,017,731).

The term "yeast" as used in the context of yeast expression systems generally refers to organisms of the order *Saccharomycetales*, such as *S. cerevisiae* and *Pichia pastoris*. For the purposes of this disclosure, the terms "fungi" and "fungal" should be understood to refer to *Basidiomycetes, Zygomycetes, Oomycetes*, and *Chythridiomycetes*, and *Ascomycetes* of the class *Euascomycetes*, which are not of the order *Saccharomycetales*. Filamentous fungi may be distinguished from yeast by their hyphal elongation during vegetative growth, and obligately aerobic carbon catabolism (vegetative growth in yeast is accomplished by budding from a unicellular thallus, and yeast may employ fermentative catabolism.)

Proper intron splicing, and glycosylation, folding, and other post-translational modifications of fungal gene products would be most efficiently handled by a fungal host species, making filamentous fungi superior hosts for screening genomic DNA from soil samples. It also makes them excellent hosts for the production of fungal enzymes of commercial interest, such as proteases, cellulases, and amylases. It has also been found that filamentous fungi are capable of transcribing, translating, processing, and secreting the products of other eukaryotic genes, including mammalian genes. The latter property makes filamentous fungi attractive hosts for the production of proteins of biomedical interest. Glycosylation patterns introduced by filamentous fungi more closely resemble those of mammalian proteins than do the patterns introduced by yeast. For these reasons, a great deal of effort has been expended on the development of fungal host systems for expression of heterologous proteins, and a number of fungal expression systems have been developed. For reviews of work in this area, see Maras et al., Glycoconjugate J.,16:99-107 (1999); Peberdy, Acta Microbiol. Immunol. Hung. 46:165-174 (1999); Kruszewsa, Acta Biochim. Pol. 46:181-195 (1999); Archer et al., Crit. Rev. Biotechnol.17:273-306 (1997); and Jeenes et al., Biotech. Genet. Eng. Rev. 9:327-367 (1991).

High-throughput expression and assaying of DNA libraries derived from fungal genomes would also be of use in assigning functions to the many mammalian genes that are currently of unknown function. For example, once a fungal protein having a property of activity of interest is identified, the sequence of the encoding gene may be compared to the human genome sequence to look for homologous genes.

Yelton et al., U.S. Pat. No. 4,816,405, discloses the modification of filamentous *Ascomycetes* to produce and secrete heterologous proteins. Buxton et al., in U.S. Pat. No. 4,885,249, and in Buxton and Radford, Mol. Gen. Genet.196:339-344 (1984), discloses the transformation of *Aspergillus niger* by a DNA vector that contains a selectable marker capable of being incorporated into the host cells. McKnight et al., U.S. patent. 4,935,349, and Boel, in U.S. patent 5,536,661, disclose methods for expressing eukaryotic genes in *Aspergillus* involving promoters capable of directing the expression of heterologous genes in *Aspergillus* and other filamentous fungi. Royer et al., in US patent 5,837,847, and Berka et al., in WO 00/56900, disclose expression systems for use in *Fusarium venenatum* employing natural and mutant *Fusarium spp.* promoters. Conneely et al., in U.S. patent 5,955,316, disclose plasmid constructs suitable for the expression and production of lactoferrin in *Aspergillus. Cladosporium* glucose oxidase had been expressed in *Aspergillus* (U.S. patent 5,879,921).

Similar techniques have been used in *Neurospora.* Lambowitz, in U.S. patent 4,486,533, discloses an autonomously replicating DNA vector for filamentous fungi and its use for the introduction and expression of heterologous genes in *Neurospora.* Stuart *et al.* describe cotransformation of *Neurospora crassa* spheroplasts with mammalian genes and endogenous transcriptional regulatory elements in U.S. patent 5,695,965, and an improved strain of *Neurospora* having reduced levels of extracellular protease in U.S. patent 5,776,730. Vectors for transformation of *Neurospora* are disclosed in U.S. patent 5,834,191. Takagi *et al.* describe a transformation system for *Rhizopus* in U.S. patent 5,436,158. Sisniega-Barroso *et al.* describe a transformation system for filamentous fungi in WO 99/51756, which employs promoters of the glutamate dehydrogenase genes from *Aspergillus awamori*. Dantas-Barbosa et al., FEMS Microbiol. Lett. 1998 169:185-190, describe transformation of *Humicola grisea* var. *thermoidea* to hygromycin B resistance, using either the lithium acetate method or electroporation.

Among the more successful fungal expression systems are those of *Aspergillus* and *Trichoderma,* for example as disclosed by Berka et al. in U.S. Patent 5,578,463; see also Devchand and Gwynne, J. Biotechnol. 17:3-9 (1991) and Gouka et al., Appl. Microbiol. Biotechnol. 47:1-11 (1997). Examples of transformed strains of *Myceliophthora thermophila, Acremonium alabamense, Thielavia terrestris* and *Sporotrichum cellulophilum* are presented in WO 96/02563 and U.S. patents 5,602,004, 5,604,129 and 5,695,985, which describe certain drawbacks of the *Aspergillus* and *Trichoderma* systems and suggest that other fungi may be more suited to large scale protein production.

Methods for the transformation of phyla other than *Ascomycetes* are known in the art; see for example Munoz-Rivas et al., Mol. Gen. Genet. 1986 205:103-106 (*Schizophyllum commune*); van de Rhee et al., Moll. Gen. Genet. 1996 250:252-258 (*Agaricus bisporus*); Aenau et al., Mol. Gen. Genet. 1991 225:193-198 (*Mucor circinelloides*); Liou et al., Biosci. Biotechnol. Biochem. 1992 56:1503-1504 (*Rhizopus niveus*); Judelson et al., Mol. Plant Microbe Interact. 1991 4:602-607 (*Phytophthora infestans*); and de Groot et al., Nature Biotechnol. 1998 16:839-842 (*Agaricus bisporus*).

In addition to the usual methods of transformation of filamentous fungi, such as for example protoplast fusion, Chakraborty and Kapoor, Nucleic Acids Res.18:6737 (1990) describe the transformation of filamentous fungi by electroporation. De Groot et al., in Nature Biotechnol. 16: 839-842 (1998), describe *Agrobacterium tumefaciens*-mediated transformation of several filamentous fungi. Biolistic introduction of DNA into fungi has been carried out; see for example Christiansen et al., Curr. Genet. 29:100-102 (1995); Durand et al., Curr. Genet. 31:158-161 (1997); and Barcellos et al., Can. J. Microbiol. 44:1137-1141 (1998). The use of magnetic particles for "magneto-biolistic" transfection of cells is described in U.S. patents 5,516,670 and 5,753,477, and is expected to be applicable to filamentous fungi.

It is evident that much work has been done to develop expression systems using fungi as hosts. However, the common fungal hosts are all filamentous fungi, which tend to form entangled mats of mycelia in unstirred cultures, and highly viscous suspension (submerged) cultures in stirred tank bioreactors. These properties of filamentous fungi also cause some problems in the industrial production of enzymes in fungal host cells. For example, high viscosity and/or the local formation of dense aggregates of mycelium, leads to difficulties in agitation, aeration, and nutrient diffusion. In general, filamentous fungi are not amenable to micropipetting of suspension cultures into microtiter plates, due to the viscosity of the cultures. Furthermore, due to the entangled mycelia, a culture of a typical filamentous fungus expressing a DNA library is not easily separated into separate clones on a large scale, which prevents evaluation of the individual genotypes as would be required in a high-throughput assay system.

Typical filamentous fungi, in the absence of constant agitation, tend to grow in the form of mats on the surface of a liquid culture medium, where they produce aerial spores. They do not generally sporulate when in submerged culture. Both of these properties present substantial obstacles to the culture of filamentous fungal clones in mircotiter plates, and to the efficient manipulation and use of such cultures for high-throughput screening. Suspended spores or other reproductively competent elements would suitable for separation and distribution into individual microtiter wells, whereas the production of aerial spores will lead to cross-contamination of microtiter wells if surface mats are allowed to form. Agitation of the medium in microtiter wells, to the extent needed to prevent mat formation, is not feasible. In addition to the problem of difficult-to-control aerial spores, surface mats interfere with light transmission, making many assays (in particular spectrophotometric absorbance assays) diffcult or impossible. Surface mats also interfere with processes such as oxygenation, reagent and nutrient addition, and pipetting.

The influence of fungal morphology on the physical properties of the culture has been recognized, and naturally-occurring strains having more favorable morphology have been identified, as described for example by Jensen and Boominathan in U.S. patent 5,695,985. Homogeneous distribution of loose mycelium, with pronounced branching, was described as a particularly desirable morphology. Schuster and Royer, in international patent application WO 97/26330 and US patent 6,184,026, suggest a similar method of identifying fungal cells having more suitable morphology for industrial production of heterologous proteins. The method comprises screening mutants of a parent fungal cell line, rather than wild-type strains, to find a specific altered morphology, transforming the mutant, and assessing whether a culture of the transformed mutant produces more heterologous protein than the parent cell line. Mutants with at least 10% greater hyphal branching are particulary claimed. The method is illustrated for strains of *Trichoderma, Fusarium* and *Aspergillus,* and is suggested to be applicable to numerous other genera.

The effect of branching frequency on culture viscosity of *Aspergillus oryzae* mutants was examined by Bocking et al., Biotechnol. Bioeng. 65:638-648 (1999); more highly branched strains exhibited lower viscosity in this study. Van Wezel et al., in PCT application WO 00/00613, describe methods for reducing the branching and/or enhancing the fragmentation of filamentous microorganisms, whereby the viscosity of the culture is reduced. The method involves transforming the microorganisms with the *SsgA* gene of *Streptomyces griseus*. The method is demonstrated in filamentous bacteria of the order *Actinomycetales*, but is stated to be applicable to filamentous fungi. Dunn-Coleman et al., in WO 00/56893, describe an HbrA2 mutant *A. nidulans*, which exhibits a hyperbranched phenotype when grown above 42 °C, and noted a linear relationship between the degree of hyphal branching and culture viscosity.

Most prior efforts in the field of filamentous fungal expression systems have been directed to the identification of strains suitable for industrial production of enzymes, and therefore attention has been focused on culture viscosity, stability of transformation, yield of heterologous protein per unit volume, and yield as a percentage of biomass. DNA libraries have been expressed in fungi; see for example Gems and Clutterbuck, Curr. Genet. 1993 24:520-524, where an *Aspergillus nidulans* library was expressed in *A* nidulans and Gems et al., Mol. Gen. Genet. 1994 242:467-471 where a genomic library from *Penicillium* was expressed in *Aspergillus*. Neither of these reports disclosed or suggested screening the expressed proteins; it was through complementation of mutant alleles in the host that the expression of genes from the DNA library was demonstrated. The complementation method requires a specific mutant host for each exogenous protein activity one wishes to detect, and does not provide a tool for general library screening.

The cloning of an *Aspergillus niger* invertase gene by expression in *Trichoderma reesei* was described by Berges et al., Curr. Genet. 1993 24:53-59. Using an *A. niger* genomic library constructed in a cosmid vector containing a selectable marker, and using as the host *T. reesei* (which is incapable of utilizing sucrose), an *A. niger* invertase gene was cloned by a sib selection procedure. Here, again, a very specific characteristic of the host was required to detect the presence of a single expressed exogenous protein, and screening of the genomic library was not disclosed or enabled.

WO93/11249 describes a method for cloning proteins in yeast hosts so that the proteins are secreted from the host cell.

WO99/32617 relates to the expression of DNA in filamentous fungal hosts in order to identify proteins of interest. The method involves propagation on solid media and is not amendable to high throughput screening.

The characteristics of a fungal host cell suitable for expression of a DNA library are different in many respects from the characteristics of hosts suitable for industrial protein manufacture. In general terms, a suitable fungal host for high-throughput screening should meet numerous criteria; among them are the following:
- The host must be transformed with high efficiency.
- The host must process intron-containing genes and carry out any necessary splicing.
- The host must post-translationally process the expressed protein so that it is produced in an active form.
- Where the library is to be assayed for a protein, the host must produce the protein in high enough yield for detection by the assay.
- The host should accept a variety of expression regulatory elements, for ease of use and versatility.
- The host should permit the use of easily-selectable markers.
- The host cell cultures should be of low viscosity.
- The host should be deficient in proteases and/or be anemable to suppression of protease expression.
- The host must permit screens for a wide variety of exogenous protein activities or properties.
- The hyphae in a culture of the host fungus should not be so entangled as to prevent the isolation of single clones, and should not be so entangled as to raise the viscosity to the point of preventing efficient transfer and replication in a miniaturized high throughput screening format (*e*.*g*. by micropipeting).
- The host should not form surface mats, but should preferentially grow as a submerged culture.
- The host should allow the efficient production of submerged spores or other propagules under the growth conditions provided in the high throughput screen..

In cases where metabolites are being screened for, it would be advantageous if the host cells secreted the metabolites into the medium, where they could be readily detected and/or assayed. Ideally, the host should secrete only the exogenous protein.

In cases where a protein is being assayed for, it would be particularly advantageous if the host also expressed enough heterologous protein to enable isolation and purification of the protein. A host cell with this characteristic would make it possible to further characterize all heterologous proteins of interest merely by culturing the host cells, without the time-consuming molecular biological manipulations neeed to transfer the gene to another organism. Preferably, the host should be capable of secretion of the protein, as this would permit more reliable and more varied assays.

It would also be advantageous if the host cell were amenable to ready isolation of the heterologous DNA, so that further studies and modifications of the gene itself may be carried out.

In addition to these qualities of the host, the transformation system should also exhibit certrain characteristics. The transformation frequency should be sufficiently high to generate the numbers of transformants required for meaningful screens. Ideally, expression of the exogenous protein will be induced by a single inducer, by a single pathway, acting on a single promoter.

To date, no combination of host cells and transformation system has been developed that meets all, or even most, of these criteria. A need therefore remains for fungal host cell and transformation systems that are capable of efficiently expressing the gene products of a DNA library, especially genomic and/or eukaryotic genomic DNA libraries.

### BRIEF DESCRIPTION OF THE INVENTION.

The present invention employs filamentous fungi which produce "transferable reproductive elements" when grown in submerged culture. By "transferable reproductive element" is meant a spore, propagule, hyphal fragment, protoplast, micropellet, or other fungal element that is (1) readily separated from other such elements in the culture medium, and (2) capable of reproducing itself into a monoclonal culture. The fungi preferably also exhibit a less pronounced filamentous phenotype and/or a compact growth morphology, and produce low-viscosity cultures that are suitable for the physical manipulations involved in high-throughput DNA library screening. Particularly preferred are filamentous fungi which, even in the absence of agitation, tend to grow as submerged cultures rather than as surface mats.

The present invention takes advantage of the properties of the transformation system disclosed in international patent applications PCT/NL99/00618 and PCT/EP99/202516. These applications describe an efficient transformation system for filamentous fungal hosts such as *Cluysosporium lucknowense and Aspergillus sojae*. These applications also disclose that mutant strains are readily prepared which retain all the advantages of the wild-type host cells, but which have partially lost their filamentous phenotype and thus provide low-viscosity cultures.

The fungi preferred for use in the invention express and secrete large amounts of exogenous protein, producing a high protein/biomass ratio relative to previously known filamentous fungal hosts. The invention provides a transformation system that exhibits high yields of transformants. The invention also provides libraries of transformant fungi which efficiently express the protein products of heterologous cDNA inserts, and especially genomic DNA inserts. In another aspect of the invention, the libraries of transformed fungi may be used in screening for activities or properties of the heterologous proteins, or in screening for metabolites produced by the transformed fungi as a consequence of exogenous protein activities, or in screening for the heterologous DNA or for RNA transcripts derived therefrom. It will be appreciated that the present invention also enables high-throughput screening for metabolites of non-transformed strains having the phenotypic characteristics described above.

The term "mutant filamentous fungus" as used herein refers simply to fungi not found in nature. The "mutations" that lead to desirable phenotypic characteristics, such as a compact growth form, low viscosity, reduced protease levels, submerged growth, etc., may be introduced randomly by either classical means, such as UV irradiation and chemical mutagenesis, or by molecular biological methods such as cassette mutagenesis, or may be deliberately introduced by genetic engineering methods. Should a naturally-occurring fungus be found to possess the necessary properties, it will of course be usable in the methods of the invention.

In yet another aspect of the invention, the libraries of transformed fungi may be screened for useful properties of the fungi themselves, such as for example high levels of production of a particular expressed protein or metabolite. This aspect of the invention is illustrated by a quantitative assay for the expressed protein of interest, where the particular transformant having the most favorable combination of protein production, protein processing, and protein secretion would be detected.

In another aspect of the invention, the libraries of transformed fungi may be screened for the presence of DNA sequences capable of hybridizing to a nucleic acid probe of interest.

### DESCRIPTION OF THE FIGURES

Figure 1 is a Western blot as described in the Examples.
Figure 2 is a pUT720 map.
Figure 3 is a pUT970G map.
Figure 4 is a pUT1064 map.
Figure 5 is a pUT1 065 map.
Figure 6 is a pF6g map.
Figure 7 is a pUT1150 map.
Figure 8 is a pUT1152 map.
Figure 9 is a pUT1155 map.
Figure 10 is a pUT1160 map.
Figure 11 is a pUT1162 map.

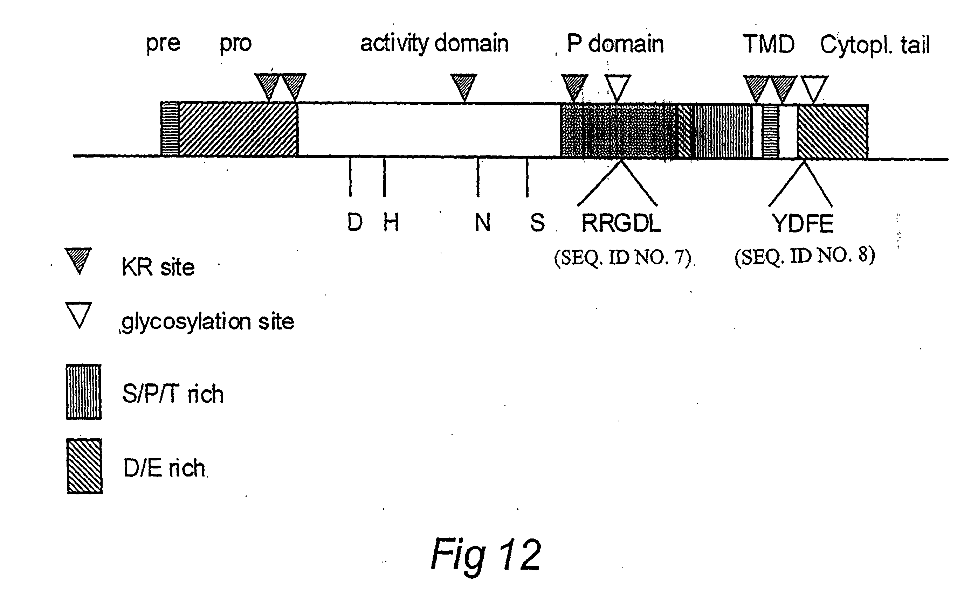

Figure 12 is the schematic structure of the pclA protein.
Figure 13A is a photomicrograph of wildtype *Aspergillus niger.*
Figure 13B is a photomicrograph of an *Aspergillus niger* pclA mutant.
Figure 14A is a photomicrograph of wildtype *Aspergillus sojae*.
Figure 14B is a photomicrobraph of an *Aspergillus sojae* pclA mutant.
Figures 15A-E present sequencing results of the pyrE gene. Underlining indicates amino acid sequence; it is not continous due to some sequence uncertainties. The indicated amino acids are the most probable. Bold type indicates putative/probable introns.

### DETAILED DESCRIPTION OF THE INVENTION.

In its broadest aspect, the invention is directed to transformed filamentous fungi that generate transferable reproductive elements in suspension, to libraries of such fungi, and to methods of screening such libraries for biological properties of interest, such as biochemical or biological activity associated with expressed exogenous proteins or associated with metabolites, i. e. small molecule products produced by endgoenous and/or exogenous enzymes. The library of low-viscosity filamentous fungi comprises fungi containing nucleic acid sequences, each nucleic acid sequence encoding a heterologous protein, each of said nucleic acid sequences being operably linked to an expression regulating region and optionally a secretion signal encoding sequence and/or a carrier protein encoding sequence. Preferably a transformed strain according to the invention will secrete the heterologous protein.

The expression and screeing methods of the invention, and the fungi employed therein, are useful for producing fungi, proteins, metabolites, and DNA molecules having utility in a variety of applications. The methods of the invention are also useful for producing nucleic acid and protein sequence information, and this information itself is regarded as a valuable product of the claimed methods.

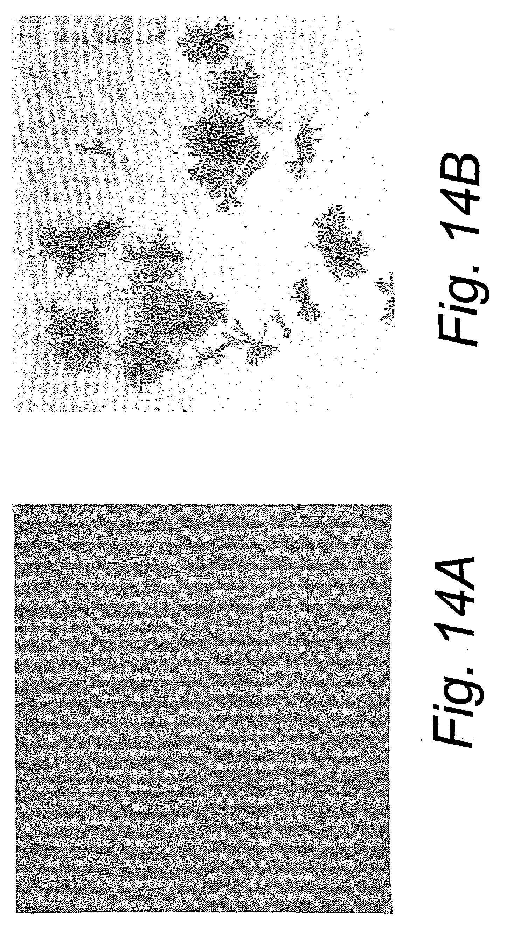

Preferred filamentous fungi of the invention are characterized by the low viscosity of the culture medium. Whereas a typical industrial-grade filamentous fungus will produce cultures with viscosities well over 200 centipoise (cP) and usually over 1,000 cP, and can reach 10,000 cP, the fungi of this invention exhibit a culture viscosity of less than 200 cP, preferably less than 100 cP, more preferably less than 60 cP, and most preferably less than 10 cP after 48 or more hours of culturing in the presence of adequate nutrients under optimal or near-optimal growth conditions. The filamentous fungi of the invention usually exhibit a morphology characterized by short, discrete, non-entangled hyphae, or micropellets. Micropellets are slightly- or non-entangled collections of hyphae arising from a single clone, as distinct from pellets which are much larger and are derived from multiple entangled clones. For example, the mutant UV 18-25 *Chrysosporium lucknowense* strain ( viscosity < 10 cP ) and the morphologically similar mutant *Trichoderma longibrachiatum* X-252 strain (viscosity < 60 cP ) are characterised by the presence of short, distinct, non-entangled hyphae between 100 and 200 microns in length, and the low viscosity engineered mutant *Aspergillus sojae* pclA is characterized by a compact form with considerable branching and short hyphae (see Fig. 14). Whereas the low-viscosity fungi described in WO97/26330 are described as having "more extensive hyphal branching," some fungi of the present invention have equivalent or even slightly reduced hyphal branching when compared to the non-mutant strains. It appears that hyphal length plays the dominant role in controlling the viscosity of the culture.

Particularly preferred fungal strains are characterized by having a high exogenous secreted protein/biomass ratio. This ratio is preferably greater than 1:1, more preferably greater than 2:1, and even more preferably 6:1 or greater. Most preferably, the ratio is 8:1 or higher. Such high ratios are advantageous in a high-throughput screening environment, because they result in a higher concentration of exogenous protein, allowing more sensitive and/or more rapid screening assays. This is of particular benefit as the volume of the assay solution decreases, for example upon going from 96-well plates to 384-well plates, and thence to 1536-well plates. The methods of the present invention are suitable for any of these microtiter plate formats, and for most other HTS formats employing liquid samples.

It is contemplated that any filamentous fungus can be converted, by the processes of mutation described herein, into mutant strains suitable for use in the present invention. Among the preferred genera of filamentous fungi are the *Chrysosporium, Thielavia, Neurospora, Aureobasidium, Filibasidium, Piromyces, Cryplococcus, Acremonium, Tolypocladium, Scytalidium, Schizophyllum, Sporotrichum, Penicillium, Gibberella, Myceliophthora, Mucor, Aspergillus, Fusarium, Humicola*, and *Trichoderma*, and anamorphs and teleomorphs thereof. More preferred are *Chrysosporium, Trichoderma, Aspergillus*, and *Fusarium*. Most preferred is *Chrysosporium*. The genus and species of fungi can be defined by morphology consistent with that disclosed in Barnett and Hunter, Illustrated Genera of Imperfect Fungi, 3rd Edition, 1972, Burgess Publishing Company. A source providing details concerning classification of fungi of the genus *Chrysosporium* is Van Oorschot, C.A.N. (1980) "A revision of Chrysosporium and allied genera" in Studies in Mycology No. 20, Centraal Bureau voor Schimmelcultures (CBS), Baam, The Netherlands, pp. 1-36. According to these teachings the genus *Chrysosporium* falls within the family *Moniliaceae* which belongs to the order *Hyphomycetales*.

Another ready source providing information on fungal nomenclature are the Budapest Treaty depositories, especially those providing online databases (the following internet addresses employ the http protocol). The ATCC (US) provides information at www.atcc.org, the CBS (NE) at www.cbs.knaw.nl, and the VKM (RU) at www.bdt.org.br.bdt.msdn.vkm/general. Another source is NT.ars-grin.gov/fungaldatabases. All these institutions can provide teaching on the distinguishing characteristics of fungal species. An alternate taxonomy of the *Ascomycota* may be found at www.ncbi.nlm.nih.gov/htbin-post/Taxonomy/wgetorg?mode=Undef&id=4890. According to this alternate taxonomy, the genus *Chrysosporium* belongs to family *Onygenaceae,* order *Onygenales*, phylum *Ascomycota*.

The definition of *Chrysosporium* includes but is not limited to these strains: *C. botryoides, C. carmichaelii, C. crassitunicatum, C. europae, C. evolceannui, C. farinicola, C. fastidium, C. filiforme, C. georgiae, C. globiferum, C. globiferum* var. *articulatum, C. globiferum* var. *niveum, C. hirundo, C. hispanicum, C. holmii, C. indicum, C. inops, C. keratinophilum, C. kreiselii, C. kuzurovianum, C. lignorum, C. lobatum, C. lucknowense, C. lucknowense* Garg 27K, *C. medium, C. medium* var. *spissescens, C. mephiticum, C. merdarium, C. merdarium* var. *roseum, C. minor, C. pannicola, C. parvum, C. parvum* var. *crescens, C. pilosum, C. pseudomerdarium, C. pyriformis, C. queenslandicum, C. sigleri, C. sulfureum, C. synchronum, C. tropicum, C. undulatum, C. vallenarense, C. vespertilium, C. zonatum.*

*C. lucknowense* is a species of *Chrysosporium* that is of particular interest as it has provided a natural high producer of cellulase proteins (international applications WO 98/15633, PCT/NL99/00618, and U.S. patents 5,811,381 and 6,015,707). Strains with international depository accession numbers ATCC 44006, CBS 251.72, CBS 143.77, CBS 272.77, and VKM F-3500D are examples of *Chrysosporium lucknowense* strains. Also included within the definition of *Chrysoporium* are strains derived from *Chrysosporium* predecessors including those that have mutated either naturally or by induced mutagenesis. The methods of the invention, in one embodiment, employ mutants of *Chrysosporium*, obtained by a combination of irradiation and chemically-induced mutagenesis, that tend to produce transferable reproductive elements in suspension, and that exhibit a morphology characterized by short, discrete, non entangled hyphae ("compact growth"), and a phenotype characterized by submerged growth and reduced viscosity of the fermentation medium when cultured in suspension. In another embodiment, the invention employs phenotypically similar mutants of *Trichoderma*. In yet other embodiments the invention employs phenotypically similar mutants of *Aspergillus sojae* or *Aspergillus niger.*

For example, VKM F-3500D (strain "C1") was mutagenised by subjecting it to ultraviolet light to generate strain UV13-6. This strain was subsequently further mutated with N-methyl-N'-nitro-N-nitrosoguanidine to generate strain NG7C-19. The latter strain in turn was subjected to mutation by ultraviolet light resulting in strain UV18-25 (VKM F-3631D). During this mutation process the morphological characteristics varied somewhat in culture in liquid or on plates as well as under the microscope. With each successive mutagenesis the cultures showed less of the fluffy and felty appearance on plates that are described as being characteristic of *Chrysosporium,* until the colonies attained a flat and matted appearance. A brown pigment observed with the wild type strain in some media was less prevalent in mutant strains. In liquid culture the mutant UV18-25 was noticeably less viscous than the wild type strain C1 and the mutants UV13-6 and NG7C-19. While all strains maintained the gross microscopic characteristics of *Chrysosporium,* the mycelia became narrower with each successive mutation and with UV 18-25 distinct fragmentation of the mycelia could be observed. This mycelial fragmentation is likely to be a cause of the lower viscosity associated with cultures of UV18-25. The capacity of the strains for aerial sporulation decreased with each mutagenic step. These results demonstrate that a strain may belong genetically to the genus *Chrysosporium* while exhibiting deviations from the traditional taxonomic (morphological) definitions.

In particular the anamorph form of *Chrysosporium* has been found to be suited for the screening application according to the invention. The metabolism of the anamorph renders it particularly suitable for a high degree of expression. A teleomorph should also be suitable as the genetic make-up of the anamorphs and teleomorphs is identical. The difference between anamorph and teleomorph is that one is the asexual state and the other is the sexual state; the two states exhibit different morphology under certain conditions.

Another example embodies genetically engineered mutant strains *ofAspergillus sojae.* In one of these mutants a specific endoprotease encoding gene was disrupted. This resulted in a compact growth phenotype exhibiting enhanced branching and short hyphae, and the formation of micropellets in submerged cultivation. Moreover, the *Aspergillus sojae* referred to in this application may be induced to exhibit efficient sporulation under specific submerged cultivation conditions, which renders it especially suitable for use in a high-throughput screening system. In this-case, the conditions conducive to formation of the transferable reproductive elements simply consisted of a synthetic medium containing 0.6 g/ml EDTA. The conducive conditions will vary from one host to another, but it is evident that the conditions will already be known if a host has been found to be suitable.

It is preferable to use non-toxigenic and non-pathogenic fungal strains, of which a number are known in the art, as this will reduce risks to the practitioner and will simplify the overall screening process. In a preferred embodiment the fungi will also be protease deficient, so as to minimize degradation of the exogenous proteins, and/or amenable to suppression of protease production. The use of protease deficient strains as expression hosts is well known; see for example PCT application WO 96/29391. Protease deficient strains may be produced by screening of mutants, or the protease gene(s) may be "knocked out" or otherwise inactivated by methods known in the art, as described for example by Christensen and Hynes in US patent 6,025,185 (*Aspergillus oryzae* with non-functional *areA* gene).

It has been found that *Chrysosporium* mutants can be made that have reduced expression of protease, thus making them even more suitable for the production of proteinaceous products, especially if the proteinaceous product is sensitive to protease activity. Thus the invention my also employ a mutant *Chrysosporium* strain which produces less protease than non-mutant *Chrysosporium* strain, for example less than *C. lucknowense* strain C1 (VKM F-3500 D). In particular the protease acitivity (other than any selective protease intended to cleave a secreted fusion protein) of such strains is less than half the amount, more preferably less than 30% of the amount, and most preferably less than about 10% the amount produced by the C1 strain. The decreased protease activity can be measured by known methods, such as by measuring the halo formed on skim milk plates or by bovine serum albumin (BSA) degradation.

It may be desirable to inactivate other genes in the host filamentous fungus, such as for example those encoding cellulases and other heavily secreted proteins, in order to minimize interference in the assay by host proteins. The genes encoding secreted proteins may be deleted or mutated, or alternatively genes controlling the induction system or other pathways involved in the expession of unwanted proteins may be modified in such a way as to reduce such expression. Where an endogenous promoter is employed in the vectors of the invention (see below), it may be especially desirable to inactivate genes for other proteins under control of the same inducer. Fungi amenable to suppression of protease secretion are those where protease expression is under the control of a regulatory element that responds to environmental conditions, such that these conditions (e.g., amino acid concentration) can be manipulated to minimize protease production.

Preferably a homologous expression-regulating region enabling high expression in the selected host is employed in the transforming vector. High expression-regulating regions derived from a heterologous host, such as from *Trichoderma* or *Aspergillus,* are well known in the art and can also be used. By way of example, and not limitation, examples of proteins known to be expressed in large quantities and thus providing suitable expression regulating sequences for use in the present invention are hydrophobin, protease, amylase, xylanase, pectinase, esterase, beta-galactosidase, cellulase (*e*.*g*. endo-glucanase, cellobiohydrolase) and polygalacturonase.

An expression-regulating region comprises a promoter sequence operably linked to a nucleic acid sequence encoding the protein to be expressed. The promoter is linked such that the positioning vis-à-vis the initiation codon of the sequence to be expressed allows expression. The promoter sequence can be constitutive but preferably is inducible. Use of an inducible promoter and appropriate induction media favors expression of genes operably linked to the promoter. Any expression regulating sequence from a homologous species, or from a heterologous strain capable of permitting expression of a protein, is envisaged. The expression regulating sequence is suitably a fungal expression-regulating region, *e.g.* an *ascomycete* regulating region. Suitably the ascomycete expression regulating region is a regulating region from any of the following genera: *Aspergillus, Trichoderma, Chrysosporium,Humicola, Neurospora, Tolypocladium, Fusarium, Penicillium, Talaromyces*, or alternative sexual forms thereof such as *Emericela* and *Hypocrea*. The cellobiohydrolase promoter from *Trichoderma*; alcohol dehydrogenase A, alcohol dehydrogenase R, glutamate dehydrogenase, TAKA amylase, glucoamylase, and glyceraldehyde phosphate dehydrogenase promoters from *Aspergillus*; phosphoglycerate and cross-pathway control promoters of *Neurospora*; lipase and aspartic proteinase promoter of *Rhizomucor miehei*; beta-galactosidase promoter of *Penicillium canescens*;and cellobiohydrolase, endoglucanase, xylanase, glyceraldehyde-3-phosphate dehydrogenase A, and protease promoters from *Chrysosporium* are representative examples. An expression regulating sequence from the same genus as the host strain is preferable, as it is more likely to be specifically adapted to the host.

Natural expression-regulating sequences from strains of *Chrysosporium* which express proteins in extremely large amounts, are particularly preferred. Examples of such strains have been deposited in accordance with the Budapest Treaty with the All Russian Collection (VKM) depository institute in Moscow. Wild type C1 strain has the number VKM F-3500 D, deposit date 29-08-1996, C1 UV13-6 mutant was deposited with number VKM F-3632 D, and deposit date 02-09-1998, C1 NG7C-19 mutant was deposited with number VKM F-3633 D and deposit date 02-09-1998 and C1 UV18-25 mutant was deposited with number VKM F-3631 D and deposit date 02-09-1998. These strains are also preferred as sources for the generation of low-viscosity mutants; indeed the VKM F-3631 D strain already exhibits the necessary low viscosity phenotype. A low-viscosity mutant *Trichoderma* strain, designated X-252, was obtained after two rounds of irradiation of *Trichoderma longibrachiatum* 18.2KK, which in turn was derived by mutation of the QM 9414 strain of *T. longibrachiatum* (ATCC 26921). In other embodiments the invention employs phenotypically similar mutants of *Aspergillus sojae* and *Aspergillus niger.*

Preferably, where the host is a *Chrysosporium*, a *Chrysosporium* promoter sequence is employed to ensure good recognition thereof by the host. Certain heterologous expression-regulating sequences also work as efficiently in *Chrysosporium* as native *Chrysosporium* sequences. This allows well-known constructs and vectors to be used in transformation of *Chrysosporium*, and offers numerous other possibilities for constructing vectors enabling good rates of transformation and expression in this host. For example, standard *Aspergillus* transformation techniques can be used as described for example by Christiansen et al. in BiolTechnology 1988 6:1419-1422. Other documents providing details of *Aspergillus* transformation vectors, *e*.*g*. US patents 4,816,405, 5,198,345, 5,503,991, 5,364,770, 5,705,358, 5,728,547, and 5,578,463, EP-B-215.594 (also for *Trichoderma*).

As extremely high expression rates for cellulase have been observed in *Chrysosporium* strains, the expression regulating regions of cellulase genes are particularly preferred.

The vectors of the invention can comprise a promoter sequence derived from a gene encoding an enzyme, preferably a secreted enzyme. Examples of suitable enzymes from which promoter sequences may be taken are the carbohydrate-degrading enzymes (e.g., cellulases, xylanases, mannanases, mannosidases, pectinases, amylases, *e.g.* glucoamylases, α-amylases, α-and β-galactosidases, α- and β-glucosidases, β-glucanases, chitinases, chitanases), proteases (endoproteases, amino-proteases, amino-and carboxy-peptidases), other hydrolases (lipases, esterases, phytases), oxidoreductases (catalases, glucose-oxidases) and transferases (transglycosylases, transglutaminases, isomerases and invertases). Several examples from *Chrysosporium lucknowense* are presented in Table A.

A nucleic acid construct will preferably comprise a nucleic acid expression regulatory region from *Chrysosporium,* more preferably from *Chrysosporium lucknowense* or a derivative thereof, operably linked to a nucleic acid sequence encoding a protein to be expressed. Particularly preferred nucleic acid constructs will comprise an expression regulatory region from *Chrysosporium* associated with cellulase or xylanase expression, preferably cellobiohydrolase expression, most preferably expression of the 55 kDa cellobiohydrolase (CBH1) described in Table A. As additional examples, the *Chrysosporium* promoter sequences of hydrophobin, protease, amylase, xylanase, esterase, pectinase, beta-galactosidase, cellulase (*e*.*g*. endoglucanase, cellobiohydrolase) and polygalacturonase are also considered to fall within the scope of the invention.

**Table A: Characteristics of selected enzymes from Chrysosporim lucknowense**

| Sample | No. of amino acids | Highest pH at which >50% activity is retained | | | Highest pH at which >70% activity is retained | | | Stability 20h, 50 °C pH 7.5/8 |
|---|---|---|---|---|---|---|---|---|
| | | CMC ase | RBB CMC ase | Other substrates | CMC ase | RBB CMC ase | Other substrates | % of max activity remaining |
| 30 Kd alkaline protease | | - | - | 12.5 | - | - | 12.0 | - |
| 30 kD Xyl (alkaline) | 333 | - | - | 10.0 | - | - | 8.5 | 80 |
| 51 kD Xyl | | - | - | 8.0 | - | - | 7.5 | - |
| 60 kD Xyl | | - | - | 9.5 | - | - | 9.0 | 85 |
| 30 kD endo (EG3) | 247 | | | | | | | |
| 45 kD endo | | 7.0 | 8.0 | - | 6.5 | 7.0 | - | 75 |
| 55 kD endo | 247 | 8.0 | 8.0 | - | 7.0 | 7.0 | - | 55 |
| 25 kD(21.8 kD)endo (EG5) | 225 | 7.5 | 10.0 | - | 6.5 | 9.0 | - | 80 |
| 43 kD(39.6 kD^{*})endo (EG6) | 395 | 8.0 | 8.0 | - | 7.2 | 7.2 | - | - |
| 45 kD α,β-Gal/β-Gluc | | - | - | 6.8 | - | - | 5.7 | - |
| 48 kD CBH | | 5.2 | 7.5 | 8.0 | 5.0 | 6.8 | - | - |
| 55 kD CBH1 | 526 | 8.0 | 9.0 | - | 7.4 | 8.5 | - | 70 |
| 65 kD PGU | | - | - | 8.0 | - | - | 7.3 | - |
| 90 kD protease | | - | - | 9.0 | - | - | 9.0 | - |
| 100 kD esterase | | - | - | 9.0 | - | - | 9.0 | - |

| | | | | | | | | |
|---|---|---|---|---|---|---|---|---|
| Notes: ^{*}molecular weights by MALDI; all others by SDS PAGE xyl = xylanase endo = endoglucanase gal = galactosidase gluc = glucosidase CBN = cellbiohydrolase PGU = polygalacturonase | | | | | | | | |

Any of the promoters or regulatory regions of expression of enzymes disclosed in Table A, for example, can be suitably employed. The nucleic acid sequences of these promoters and regulatory regions can readily be obtained from a *Chrysosporium* strain. Methods by which promoter sequences can be determined are numerous and well known in the art. Promoter sequences are generally found immediately preceding the ATG start codon at the beginning of the relevant gene. For example, promoter sequences can be identified by deleting sequences upstream of the relevant gene, using recombinant DNA techniques, and examining the effects of these deletions on expression of the gene. Also, for example, promoter sequences can often be inferred by comparing the sequence of regions upstream of the relevant gene with concensus promoter sequences.

For example, the promoter sequences of C1 endoglucanases were identified in this manner (see PCT/NL99/00618) by cloning the corresponding genes. Preferred promoters according to the invention are the 55 kDa cellobiohydrolase (CBH1), glyceraldehyde-3-phosphate dehydrogenase A, and the 30 kDa xylanase (XylF) promoters from *Chrysosporium,* as these enzymes are expressed at high level by their own promoters. The promoters of the carbohydrate-degrading enzymes of *Chrysosporium lucknowense* in particular, especially *C. lucknowense* GARG 27K, can advantageously be used for expressing libraries of proteins in other fungal host organisms.

Particular embodiments of nucleic acid sequences according to the invention are known for *Chrysosporium, Aspergillus* and *Trichoderma.* Promoters for *Chrysosporium* are described in PCT/NL99/00618. The prior art provides a number of expression regulating regions for use in *Aspergillus, e.g.* U.S. patents 4,935,349; 5,198,345; 5,252,726; 5,705,358; and 5,965,384; and PCT application WO 93107277. Expression in Trichoderma is disclosed in U.S. patent 6,022,725.

The hydrophobin gene is a fungal gene that is highly expressed. It is thus suggested that the promoter sequence of a hydrophobin gene, preferably from *Chrysosporium,* may be suitably applied as expression regulating sequence in a suitable embodiment of the invention. *Trichoderma reesei* and *Trichoderma harzianum* gene sequences for hydrophobin have been disclosed for example in the prior art as well as a gene sequence for *Aspergillus fumigatus* and *Aspergillus nidulans* and the relevant sequence information is hereby incorporated by reference (Nakari-Setala et al., Eur. J. Biochem. 1996, 235:248-255; Parta et al., Infect. Immun. 1994 62:4389-4395; Munoz et al., Curr. Genet. 1997, 32:225-230; and Stringer et al., Mol. Microbiol. 1995 16:33-44). Using this sequence information a person skilled in the art can obtain the expression regulating sequences of *Chrysosporium* hydrophobin genes without undue experimentation following standard techniques such as those suggested above. A recombinant *Chrysosporium* strain according to the invention can comprise a hydrophobin-regulating region operably linked to the sequence encoding the heterologous protein.

An expression regulating sequence can also additionally comprise an enhancer or silencer. These are also well known in the prior art and are usually located some distance away from the promoter. The expression regulating sequences can also comprise promoters with activator binding sites and repressor binding sites. In some cases such sites may also be modified to eliminate this type of regulation. For example, filamentous fungal promoters in which creA sites are present have been described. The creA sites can be mutated to ensure that the glucose repression normally resulting from the presence of creA is eliminated. Use of such a promoter enables production of the library of proteins encoded by the nucleic acid sequences regulated by the promoter in the presence of glucose. The method is exemplified in WO 94/13820 and WO 97/09438. These promoters can be used either with or without their creA sites. Mutants in which the creA sites have been mutated can be used as expression regulating sequences in a recombinant strain according to the invention and the library of nucleic acid sequences it regulates can then be expressed in the presence of glucose. Such *Chrysosporium* promoters ensure derepression in an analogous manner to that illustrated in WO 97/09438. The identity of creA sites is known from the prior art. Alternatively, it is possible to apply a promoter with CreA binding sites that have not been mutated in a host strain with a mutation elsewhere in the repression system e.g. in the creA gene itself, so that the strain can, notwithstanding the presence of creA binding sites, produce the library of proteins in the presence of glucose.

Terminator sequences are also expression-regulating sequences and these are operably linked to the 3' termini of the sequences to be expressed. A variety of known fungal terminators are likely to be functional in the host strains of the invention. Examples are the *A. nidulans* trpC terminator, *A. niger* alpha-glucosidase terminator, *A. niger* glucoamylase terminator, *Mucor miehei* carboxyl protease terminator (see US 5,578,463), and the *Trichoderma reesei* cellobiohydrolase terminator. *Chrysosporium* terminator sequences, *e*.*g*. the EG6 terminator, will of course function well in *Chrysosporium.*

A suitable transformation vector for use according to the invention may optionally have the exogenous nucleic acid sequences to be expressed operably linked to a sequence encoding a signal sequence. A signal sequence is an amino acid sequence which, when operably linked to the amino acid sequence of an expressed protein, enables secretion of the protein from the host organism. Such a signal sequence may be one associated with a heterologous protein or it may be one native to the host. The nucleic acid sequence encoding the signal sequence must be positioned in frame to permit translation of the signal sequence and the heterologous proteins. Signal sequences will be particularly preferred where the invention is being used in conjunction with directed molecular evolution, and a single, secreted exogenous protein is being evolved.

It will be understood that it is less advanatageous to incorporate a signal sequence in a vector that is to be used to express a library, as this will decrease the probability of expressing the protein of interest. In a genomic library prepared by randomly shearing the DNA and cloning into a vector, the probability that one would obtain an in frame fusion of a gene in the library to the signal sequence is low. Also, even where an in-frame fusion has been obtained, the chosen signal sequence may not work with all genes. For these reasons it may be preferable not to employ a signal sequence when screening a genomic DNA library, but rather to screen for the activity or presence of intracelllular exogenous protein. Analysis of the activity or presence of intracellular proteins may be accomplished by pretreating the transformant library with enzymes that convert the fungal cells to protoplasts, followed by lysis. The procedure has been described by van Zeyl et al., J. Biotechnol. 59:221-224 (1997). This procedure has been applied to *Chrysosporium* to allow colony PCR from *Chrysosporium* transformants grown in microtiter plates.

Any signal sequence capable of permitting secretion of a protein from a *Chrysosporium* strain is envisaged. Such a signal sequence is preferably a fungal signal sequence, more preferably an *Ascomycete* signal sequence. Suitable signal sequences can be derived from eukaryotes generally, preferably from yeasts or from any of the following genera of fungi: *Aspergillus, Trichoderma, Chrysosporium, Pichia, Neurospora, Rhizomucor, Hansenula, Humicola, Mucor, Tolypocladium, Fusarium, Penicillium, Saccharomyces, Talaromyces* or alternative sexual forms thereof such as *Emericella* and *Hypocrea*. Signal sequences that are particularly useful are those natively associated with cellobiohydrolase, endoglucanase, beta-galactosidase, xylanase, pectinase, esterase, hydrophobin, protease or amylase. Examples include amylase or glucoamylase of *Aspergillus* or *Humicola*, TAKA amylase of *Aspergillus oryzae*, α-amylase of *Aspergillus niger,* carboxyl peptidase of *Mucor* (US 5,578,463), a lipase or proteinase from *Rhizomucor miehei*, cellobiohydrolase of *Trichoderma,* beta-galactosidase of *Penicillium canescens* CBH1 from *Chrysosporium*, and the alpha mating factor of *Saccharomyces*.

Alternatively the signal sequence can be from an amylase or subtilisin gene of a strain of *Bacillus*. A signal sequence from the same genus as the host strain is extremely suitable as it is most likely to be specifically adapted to the specific host; thus when *Chrysosporium lucknowense* is the host, the signal sequence is preferably a signal sequence of *Chrysosporium. Chrysosporium* strains C1, UV13-6, NG7C-19 and UV18-25 secrete proteins in extremely large amounts, and signal sequences from these strains are of particular interest. Signal sequences from filamentous fungi and yeast may be useful, as well as signal sequences of non-fungal origin.

A transformed recombinant host fungus according to any of the embodiments of the invention can further comprise a selectable marker. Such a selectable marker permits selection of transformed or transfected cells. A selectable marker often encodes a gene product providing a specific type of resistance foreign to the non-transformed strain. This can be resistance to heavy metals, antibiotics or biocides in general. Prototrophy is also a useful selectable marker of the non-antibiotic variety. Auxotrophic markers generate nutritional deficiencies in the host cells, and genes correcting those deficiencies can be used for selection. Examples of commonly used resistance and auxotrophic selection markers are amdS (acetamidase), hph (hygromycin phosphotransferase), pyrG (orotidine-5'-phosphate decarboxylase), and pyrE (orotate P-ribosyl transferase, trpC (anthranilate synthase), argB (ornithine carbamoyltransferase), sC (sulphate adenyltransferase), bar (phosphinothricin acetyltransferase), niaD (nitrate reductase), Sh-ble (bleomycin-phleomycin resistance), mutant acetolactate synthase (sulfonylurea resistance), and neomycin phosphotransferase (aminoglycoside resistance). A preferred selection marker in *Chrysosporium* is orotate P-ribosyl transferase. Selection can be carried out by cotransformation where the selection marker is on a separate vector or where the selection marker is on the same nucleic acid fragment as the protein-encoding sequence for the heterologous protein.

A further improvement of the transformation frequency may be obtained by the use of the AMA1 replicator sequence, which is useful for example in *Aspergillus niger* (Verdoes et al., Gene 146:159-165 (1994)). This sequence results in a 10- to 100-fold increase in the transformation frequency in a number of different filamentous fungi. Furthermore, the introduced DNA is retained autonomously in the fungal cells, in a multiple-copy fashion, without integration into the fungal genome. This is expected to be beneficial for the high throughput screening method of the present invention, as the non-integrative state reduces variations in the level of gene expression between different transformants. Moreover, as the introduced DNA is not recombined into the host DNA, no unwanted mutations in the host genome will occur. Uniform levels of exogenous gene expression may be obtained by use of autonomously replicating vectors such as AMA1, or alternatively, autonomous replication in fungi can be promoted by telomeric sequences (see *e*.*g*. A. Aleksenko and L. Ivanova, *Mol. Gen. Genet*. 1998 **260**:159-164.)

As used herein the term "heterologous protein" is a protein or polypeptide not normally expressed or secreted by the host strain used for expression according to the invention. A heterologous protein may be of prokayotic origin, or it may be derived from a fungus, plant, insect, or higher animal such as a mammal. For pharmaceutical screening purposes quite often a preference will exist for human proteins, thus a preferred embodiment will be a host wherein the DNA library is of human origin. Such embodiments are therefore also considered suitable examples of the invention.

Expression of a library of human genes, derived from a genomic human DNA library, in the filamentous fungi of the invention is expected to be efficient for several reasons. It is now known that the average size of human genes is 3,000-5,000 bp, and that human introns average about 75 to about 150 bp (total range 40 - >50,000). Filamentous fungi have introns of 40-75 bp, but they can deal with introns up to 500 bp in length. On average, human genes carry 3-5 introns per gene (M. Deutsch, M. Long, Nucl. Acids Res. 1999 27:3219-3228; Table B). Human signal sequences are also known to function in filamentous fungi. For these reasons, it is likely that a large number of human genes can be expressed and secreted at high levels by the methods of this invention.

**Table B**

| **Organism** | **Introns per gene** | **Average intron size (nt) (range)** | **Intron structure** |
|---|---|---|---|
| Animal/Plant | 3-5 | 75-150 | GTnnGt.....CtxAC.....yAG |
| | | (40->50000) | |
| | | 80% under 150 nt | |
| Fungi | 3 | 40-75 | GTAnGy.....CtxAC.....yAG |
| | | (40-500) | |
| Yeast | 0.01 | 50-60 | GTATGT..TACTAAC..yAG |
| | | (?-?) | |

The methods of the invention are thus expected to be useful for expression of DNA libraries derived from both prokaryotic and eukaryotic genomes. As described above, the methods are capable of expression and discovery of both secreted and intracellular proteins, giving ready access to an extemely large number of genes and proteins.

A further aspect of the invention includes the construction and screening of fungal mutant libraries, and fungal mutant libraries prepared by the methods disclosed herein. The libraries may be obtained by transformation of the fungal hosts according to this invention with any means of integrative or non-integrative transformation, using methods known to those skilled in the art. This library of fungi based on the preferred host strains may be handled and screened for desired properties or activities of exogenous proteins in miniaturized and/or high-throughput format screening methods. By property or activity of interest is meant any physical, physicochemical, chemical, biological, or catalytic property, or any improvement, increase, or decrease in such a property, associated with an exogenous protein of a library member. The library may also be screened for metabolites, or for a property or activity associated with a metabolite, produced as a result of the presence of exogenous and/or endogenous proteins. The library may also be screened for fungi producing increased or decreased quantities of such protein or metabolites.

In another aspect of this invention, the library of transformed fungi may be screened for the presence of fungal metabolites having desirable properties. Examples of such metabolites include polyketides, alkaloids, and terpenoid natural products. It is anticipated that multiple genes or gene clusters (operons) may be transferred to the host cells of the invention, and that non-protein products generated by the action of the encoded enzymes will then be generated in the host cells. For example, it has been shown that DNA encoding the proteins necessary for production of lovastatin can be transferred to *Aspergillus oryzae* (U.S. patent 5,362,638; see also U.S. patent 5,849,541).

In another emodiment of the invention, the library of transformed fungi may be screened for the presence of DNA that hybridizes to a nucleic acid probe of interest. In this embodiment, expression and/or secretion of exogenous proteins is not essential, although it will often still be desirable. Where protein expressin is not needed, it will be appreciated that regulatory sequences are not needed in the vector.

In yet another embodiment of the invention, the library of transformed fungi may be screened for some desirable property of the fungi themselves, such as for example tolerance to a physically or chemically extreme environment, or the ability to produce, modify, degrade or metabolize a substance of interest. Such desirable properties may or may not be ascribable to the presence of a single exogenous protein. This embodiment will be of particular utility when employed as part of a process of directed evolution.

The heterologous DNA may be genomic DNA or cDNA, prepared from biological specimens by methods well known in the art. The biological specimen may be an environmental sample (for example, soil, compost, forest litter, seawater, or fresh water), or an extracted, filtered, or centrifuged or otherwise concentrated sample therefrom. Mixed cultures of microorganisms derived from environmental samples may be employed as well. The biological sample may also be derived from any single species of organism, such as a cultured microorganism, or plant, insect, or other animal such as a mammal. In addition, the heterologous DNA may be synthetic or semi-synthetic, for example random DNA sequences or DNA comprising naturally-occurring segments which have been shuffled, mutated, or otherwise altered. An example of a semi-synthetic nucleic library is found in Wagner et al., WO 00/0632. DNA from environmental samples (or mixed cultures derived therefrom) will be advantageous for the discovery of novel proteins, while the use of DNA from a single species will be advantageous in that (1) an appropriate vector may be more judiciously chosen, and (2) the practitioner will be directed to related or similar species for further screening if a protein of interest is identified.

Compared to traditional fungal hosts, transformation, expression and secretion rates are exceedingly high when using a *Chrysosporium* strain exhibiting the compact mycelial morphology of strain UV 18-25. Thus a recombinant strain according to the invention will preferably exhibit such morphology. The invention however also covers non-recombinant strains or otherwise engineered strains of fungi exhibiting this characteristic. An attractive embodiment of the invention would employ a recombinant *Chrysosporium* strain exhibiting a viscosity below that of strain NG7C-19, preferably below that of UV18-25 under corresponding or identical culture conditions. We have determined that the viscosity of a culture of UV18-25 is below 10 cP as opposed to that of previously known *Trichoderma reesei* being of the order 200-600 cP, and with that of traditional *Aspergillus niger* being of the order 1500-2000 cP under optimal culture conditions during the middle to late stages of fermentation. Accordingly the invention may employ any engineered or mutant filamentous fungus exhbiting this low-viscosity charactersistic, such as the Chrysosporium UV 18-25 (VKM F-3631D) strain, the Trichoderma X 252 strain, or A. *sojae* pclA (derived from ATCC 11906) or *A. niger* pclA.

The fluidity of filamentous fungal cultures can vary over a wide range, from nearly solid to a free-flowing liquid. Viscosity can readily be quantitated by Brookfield rotational viscometry, use of kinematic viscosity tubes, falling ball viscometer or cup type viscometer. Fermentation broths are non-Newtonian fluids, and the apparent viscosity will be dependent to some extent upon the shear rate (Goudar et al., Appl. Microbiol. Biotechnol. 1999 51:310-315). This effect is however much less pronounced for the low-viscosity cultures employed in the present invention.

The use of such low viscosity cultures in the screening of an expression library according to the method of the invention is highly advantageous. The screening of DNA libraries expressed in filamentous fungi has heretofore been limited to relatively slow and laborious methods. In general, once fungi have been transformed (and the transformants optionally selected for), it has been necessary to prepare spores or conidia, or to mechanically disrupt the mycelia, in order to disperse the library of transformed fungi into individual organisms or reproductive elements. This dispersal is necessary so that the separated organisms can be cultured into clonal colonies or cultures. The spores, conidia, or mycelial fragments are then diluted and "plated out" in standard culture dishes, and the individual colonies are inspected for color, alterations to the substrate, or other detectable indication of the presence of the protein activity or property being sought. In another approach, secreted proteins are blotted from the colonies onto a membrane, and the membrane is probed or examined for an indication of the presence of the protein activity or property of interest. Use of membranes has proved useful where proteolytic degradation of exogenous protein is a problem (Asgeirsdottir et al., Appl. Environ. Microbiol. 1999, 65:2250-2252). Such procedures are labor-intensive and have not proven amenable to automation, and as a result high-throughput screening of fungally-expressed proteins has not heretofore been accomplished with conventional filamentous fungi. For purposes of this disclosure, high-throughput screening refers to any partially- or fully-automated screening method that is capable of evaluating the proteins expressed by about 1,000 or more transformants per day, and particularly to those methods capable of evaluationg 5,000 or more transformants per day, and most particularly to methods capable of evaluating 10,000 or more transformants per day.

The automated high-throughput screening of a library of transformed fungi according to the present invention, accordingly, may be carried out in a number of known ways. Methods that are known to be applicable to bacteria or yeast may in general be applied to the low-viscosity fungi of the present invention. This is made possible by the presence of transferable reproductive elements in combination with the low-viscosity phenotype, a consequence of the relatively non-entangled morphology of the hyphae of the mutant fungi employed. In essence, the mutant fungi, and/or their transferable reproductive elements, behave very much like individual bacteria or yeast during the mechanical manipulations involved in automated high-throughput screening. This is in contrast to wild-type fungi, and most industrially-adapted fungi as well, which produce highly entangled mycelia which do not permit the ready separation of the individual organisms from one another.

For example, a dilute suspension of transformed fungi according to the present invention may be aliquotted out through a mechanical micropipette into the wells of a 96-well microplate. It is anticipated that liquid-handling apparatus capable of pipetting into 384- or 1536-well microplates can also be adapted to the task of automated dispersal of the organisms into microplates. The concentration of the suspended organisms can be adjusted as desired to control the average number of organisms (or other transferable reproductive elements) per well. It will be appreciated that where multiple individual organisms are aliquotted into wells, the identification of the desired protein activity or property in that well will be followed by dilution of the contents of the well and culturing the organisms present into individual clonal colonies or cultures. In this manner the throughput of the system may be increased, at the cost of the need for subsequent resolution of the contents of each well that presents a "hit".

In an alternative embodiment, a cell sorter may be interposed in the fluid path, which is capable of directing the flow of the culture to the wells of the microplate upon the detection of an organism or other transferable reproductive element in the detector cell. This embodiment permits the reasonably accurate dispensation of one organism per well. The use of an optically-detectable marker, such as green fluorescent protein, to identify transformats is particularly useful in this embodiment, as it permits the automated selection of transformants by a fluorescence-activated cell sorter.

In yet another embodiment, colonies growing on solid media can be picked by a robotic colony picker, and the organisms transferred by the robot to the wells of a microtiter plate. Well-separated colonies will give rise to single clones in each well.

The dispersed organisms are then permitted to grow into clonal cultures in the microplate wells. Inducers, nutrients, etc. may be added as desired by the automated fluid dispensing system. The system may also be used to add any reagents required to enable the detection of the protein activity or property of interest. For example, colorogenic or fluorogenic substrates can be added so as to permit the spectroscopic or fluorometric detection of an enzyme activity. The low viscosity and submerged growth habit of the cultures in the wells of a microtiter plate permit the rapid diffusion of such reagents into the culture, greatly enhancing the sensitivity and reliability of the assay. Diffusion of oxygen and nutrients is also greatly enhanced, facilitating rapid growth and maximal expression and secretion of exogenous peptides. Certain assays, such as the scintillation proximity assay, rely on the diffusion of soluble components so as to arrive at an equilibrium state; again the low viscosity of the fungal cultures of the present invention makes this high throughput assay possible. Finally, in a highly automated system it will be desirable to automatically pick, aspirate, or pipette clonal cultures of interest from their wells in the microtiter plate, and the low viscosity and submerged growth habit of the cultures will make this possible. All of the above operations would be difficult or impossible given the viscosity of traditional filamenous fungal cultures, especially cultures growing as surface mats in the unstirred, shear-free conditions of a microtiter plate well.

In another emodiment, single cells are passed through a microfluidic apparatus, and the property or activity of interest is detected optically (Wada et al., WO 99/67639). Low viscosity is essential to the operation of a microfluidics device, and cultures of the low-viscosity mutant fungi of the present invention are expected to be amenable to microfluidic manipulation. Short et al., in US patent 6,174,673, have described how fluorogenic substrates may be employed to detect an enzyme activity of interest, and how host cells expressing such an activity may be isolated with a fluorescence-activated cell sorter. The methods of the present invention are compatible with this method of identification of expressed proteins.

In one embodiment, where transformants carry a fluorescent protein as a marker, the fluorescence may be quantitated and employed as a measure of the amount of gene expression and/or expressed protein present in a given culture. In this embodiment, it is possible not only to detect an exogenous protein of interest, but to estimate the specific activity of the protein, as described by Blyna et al. in WO 00/78997. This embodiment will be particularly preferred where the screening method of the invention is employed as part of a process of directed evolution.

In those cases where a greater viscosity is acceptable, a gel-forming matrix may provide certain advantages when culturing fungi, and conducting biochemical assays, in a microplate format, as described by Bochner in US patent 6,046,021.

Another class of high-though put screens is by photometric analysis, by digital imaging spectroscopy, of large numbers of individual colonies growing on a solid substrate. See for example Youvan *et al*., 1994, *Meth. Enzymol*. **246**:732-748. In this method, changes in the overall absorption or emission spectra of specialized reagents are indicative of the presence of a heterologous protein activity or property of interest. The ready dispersal of individual organisms attendant upon the use of low-viscosity mutants also enables the use of filamentous fungi in this method. The tendency for colonies of the mutant fungi of the invention to exhibit less lateral growth, and to produce smooth, compact, and well-defined colonies on solid media, is also advantageous in such a screening system. Furthermore, the superior expression and secretion characteristics of fungi as compared to bacteria provide greater quantities of protein for spectral analysis.

An automated microorganism handling tool is described in Japanese patent application publication number 11-304666. This device is capable of the transfer of microdroplets containing individual cells, and it is anticipated that the fungal strains of the present invention, by virtue of their morphology, will be amenable to micromanipulation of individual clones with this device.

An automated microbiological high-throughput screening system is described in Beydon *et al., J. Biomol. Screening* **5**:13-21 (2000). The robotic system is capable of transferring droplets with a volume of 400 nl to agar plates, and processing 10,000 screening points per hour, and has been used to conduct yeast two-hybrid screens. It is anticipated that the fungal hosts of the present invention will be as amenable as yeast to high-throughput screening with systems of this type.

As an alternative to microtiter plates, transformants can be grown on plates and, in the form of microcolonies, assayed optically as described in WO 00/78997.

The development of high throughput screens in general is discussed by Jayawickreme and Kost, Curr. Opin. Biotechnol. 8:629-634 (1997). A high throughput screen for rarely transcribed differentially expressed genes is described in von Stein et al., Nucleic Acids Res. 35: 2598-2602 (1997).

The *Chrysosporium* strain LTV1 8-25 and the *Trichoderma* strain X 252 illustrate various aspects of the invention exceedingly well. The invention however may employ other mutant or otherwise engineered strains of filamentous fungi that produce transferable reproductive elements in suspension and exhibit low viscocity in culture. The specific morphology of the fungi may not be critical; the present inventors have observed short, non-entangled mycelia in these two strains but other morphologies, such as close and extensive hyphal branching, may also lead to reduced viscosity. Fungal strains according to the invention are preferred if they exhibit optimal growth conditions at neutral pH and temperatures of 25-43 °C. Such screening conditions are advantageous for maintaining the activity of exogenous proteins, in particular those susceptible to degradation or inactivation at acidic pH. Most mammalian proteins, and human proteins in particular, have evolved to function at physiological pH and temperature, and screening for the normal activity of a human enzyme is best carried out under those conditions. Proteins intended for therapeutic use will have to function under such conditions, which also makes these the preferred screening conditions. *Chrysosporium* strains exhibit precisely this characteristic, growing well at neutral pH and 35-40 °C, while other commonly employed fungal host species (e.g. *Aspergillus* and *Trichoderma*) grow best at acidic pH and may be less suitable for this reason.

Another application of the method of the present invention is in the process of "directed evolution," wherein novel protein-encoding DNA sequences are generated, the encoded proteins are expressed in a host cell, and those seqences encoding proteins exhibiting a desired characteristic are selected, mutated, and expressed again. The process is repeated for a number of cycles until a protein with the desired characteristics is obtained. Gene shuffling, protein engineering, error-prone PCR, site-directed mutagenesis, and combinatorial and random mutagenesis are examples of processes through which novel DNA sequences encoding exogenous proteins can be generated. U.S. patents 5,223,409, 5,780,279 and 5,770,356 provide teaching of directed evolution. See also Kuchner and Arnold, Trends in Biotechnology, 15:523-530 (1997); Schmidt-Dannert and Arnold, Trends in Biotech., 17:135-136 (1999); Arnold and Volkov, Curr. Opin. Chem. Biol., 3:54-59 (1999); Zhao et al., Manual of Industrial Microbiology and Biotechnology, 2nd Ed., (Demain and Davies, eds.) pp. 597-604, ASM Press, Washington DC, 1999; Arnold and Wintrode, Encyclopedia of Bioprocess Technology: Fermentation, Biocatalysis, and Bioseparation, (Flickinger and Drew, eds.) pp. 971-987, John Wiley & Sons, New York, 1999; and Minshull and Stemmer, Curr. Open. Chem. Biol. 3:284-290.

An application of combinatorial mutagenesis is disclosed in Hu et al., Biochemistry. 1998 37:10006-10015. US 5,763,192 describes a process for obtaining novel protein-encoding DNA sequences by stochastically generating synthetic sequences, introducing them into a host, and selecting host cells with the desired characteristic. Methods for effecting artificial gene recombination (DNA shuffling) include random priming recombination (Z. Shao, et al., Nucleic Acids Res., 26:681-683 (1998)), the staggered extension process (H. Zhao et al., Nature Biotech., 16:258-262 (1998)), and heteroduplex recombination (A. Volkov et al., Nucleic Acids Res., 27:e18 (1999)). Error-prone PCR is yet another approach (Song and Rhee, Appl. Environ. Microbiol. 66:890-894 (2000)).

There are two widely-practiced methods of carrying out the selection step in a directed evolution process. In one method, the protein activity of interest is somehow made essential to the survival of the host cells. For example, if the activity desired is a cellulase active at pH 8, a cellulase gene could be mutated and introduced into the host cells. The transformants are grown with cellulose as the sole carbon source, and the pH raised gradually until only a few survivors remain. The mutated cellulase gene from the survivors, which presumably encodes a cellulase active at relatively high pH, is subjected to another round of mutation, and the process is repeated until transformants that can grow well on cellulose at pH 8 are obtained. Thermostable variants of enzymes can likewise be evolved, by cycles of gene mutation and high-temperature culturing of host cells (Liao et al., Proc. Natl. Acad. Sci. USA 1986 83:576-580; Giver et al., Proc. Natl. Acad. Sci. U S A. 1998 95:12809-12813. For purposes of this application, mutation of DNA sequences encoding exogenous proteins may be accomplished by any of several methods employed for directed evolution, for example by gene shuffling, in vivo recombination, or cassette mutagenesis.

The chief advantage of this method is the massively parallel nature of the "survival of the fittest" selection step. Millions, or billions, of unsuccessful mutations are simultaneously eliminated from consideration without the need to evaluate them individually. However, it is not always possible to link an enzyme activity of interest to the survival of the host. For example where the desired protein property is selective binding to a target of interest, making the binding property essential to survival is likely to be difficult. Also, survival under forced conditions such as high temperature or extreme pH is likely to be dependent upon multiple factors, and a desirable mutation will not be selected for and will be lost if the host cell is unable to survive for reasons unrelated to the properties of the mutant protein.

An alternative to the massively parallel "survival of the fittest" approach is serial screening. In this approach, individual transformants are screened by traditional methods, such as observation of cleared or colored zones around colonies growing on indicator media, colorimetric or fluorometric enzyme assays, immunoassays, binding assays, etc. See for example Joo et al., Nature 399:670-673 (1999), where a cytochrome P450 monooxygenase not requiring NADH as a cofactor was evolved by cycles of mutation and screening; May et al., Nature Biotech. 18:317-320 (2000), where a hydantoinase of reversed stereoselectivity was evolved in a similar fashion; and Miyazaki et al., J. Mol. Biol. 297:1015-1026 (2000), where a thermostable subtilisin was evolved.

The screening approach has clear advantages over a simple "survival screen," especially if it can be carried out in a high-throughput manner that approaches the throughput of the massively parallel "survival screen" technique. For example, a degree of parallelism has been introduced by employing such measures as digital imaging of the transformed organisms (Joo et al., Chemistry & Biology, 6:699-706 (1999)) or digital spectroscopic evaluation of colonies (Youvan et al., 1994, Meth. Enzymol. 246:732-748). Serial assays can be automated by the use of cell sorting (Fu et al., Nature Biotech., 17:1109-1111 (1999)). A well-established approach to high-thorughput screening involves the automated evaluation of expressed proteins in microtiter plates, using commercially available plate readers, and the method of the present invention is well-suited to the application of this mode of high-throughput screening to directed evolution.

In this embodiment of the invention, a gene encoding a protein of interest is mutated by any known method of generating a plurality of mutants, the mutant protein-encoding DNA is introduced by means of a suitable expression vector into a low-viscosity filamentous fungal host according to the present invention, and the transformants are optionally selected for and cultured. The host cells are then dispersed as described previously into the wells of a microtiter plate, or otherwise spatially separated into resolvable locations, so as to provide individual monoclonal cultures (or poly-clonal cultures having fewer than about 100 diferent clones). The cells are preferably dispersed into the wells of a micro-titer plate. The protein encoded by the mutant DNA is preferably secreted into the medium in the wells of the microtiter plates. Each of the dispersed cultures is screened for the protein activity of interest, and those most strongly exhibiting the desired property are selected. The gene encoding the protein of interest in the selected cultures is mutated again, the mutant DNA is again introduced into the low-viscosity fungal host, and the transformants are re-screened. The mutating and re-screening process is repeated until the value of the property of interest reaches a desired level.

In an alternative embodiment, directed evolution is carried out by mutation and reproduction of the gene of interest in another organism, such as *E. coli,* followed by transfer of the mutant genes to a filamentous fungus according to the present invention for screening.

It will be readily appreciated by those skilled in the art that a protein that appears to be of interest based upon the screening assay will not necessarily have all the other properties required for commercial utility. For example, the possession of enzymatic activity, however high the specific activity, will not indicate that the mutant enzyme has the requisite thermal or pH stability, or detergent or protease resistance, or non-immunogenicity, or other property that might be desirable or necessary in a commercially viable product. There is a need for methods of readily determining whether an identified protein has commercially useful properties.

The prior art approaches to screening have not provided a solution to this need, because the host organisms (bacteria and yeast) were not adapted to the production of isolable quantities of protein. It has heretofore been necessary to transfer potentially useful genes from one organism to another, as one proceeded through DNA library preparation, gene expression, screening, expressionof research quantities of gene products, and over-expression in industrially suitable production strains. The mutant filamentous fungi of the present invention, on the other hand, are excellent overproducers and secretors of exogenous proteins, especially when employed with the vectors disclosed herein. Sufficient protein may be isolated not only for purposes of characterization, but for evaluation in application trials. Indeed, the strains used in the screening method of the invention are suitable for industrial production as well, since they possess desirable production properties such as low viscosity, high expression rates, and very high protein/biomass ratios.

Accordingly, in a preferred embodiment of the present invention, the method further comprises culturing a clonal colony or culture identified according to the method of the invention, under conditions permitting expression and secretion of the exogenous library protein (or a precursor thereof), and recovering the subsequently produced protein to obtain the protein of interest. Expression and secretion of a library protein may be facilitated by creating an in-frame fusion of the cloned gene with the gene for a heterologous protein (or a fragment thereof) with its corresponding signal sequence, or with the signal sequence from a third protein, all operably linked to an expression regulating sequence. By this approach a fusion protein is created that contains heterologous amino acid sequences upstream of the library protein. Subsequently, this fusion precursor protein may be isolated and recovered using purifaction techniques known in the art. The method may optionally comprise subjecting the secreted fusion protein precursor to a cleavage step to generate the library protein of interest. The cleavage step can be carried out with Kex-2, a Kex-2 like protease, or another selective protease, when the vector is engineered so that a protease cleavage site links a well-secreted protein carrier and the protein of interest.

The ready availability of mutant protein, directly from the screening host organism, has not previously been possible with prior art screening hosts. The present invention thus provides an advantage, in that the mutant proteins deemed of interest based upon the high-throughput screen can be isolated in sufficient quantities (milligrams) for further characterization and even larger quantities (grams to kilograms) for application trials. This particular embodiment of the invention thus permits the practitioner to select mutant proteins for the next round of directed evolution based upon any number of desirable properties, and not merely upon the one property detected in the high-throughput screen. The more stringent selection criteria made possible by the present invention should lead to a more efficient and cost-effective directed evolution process.

The method of production of a recombinant mutant filamentous fungal strain according to the invention comprises introducing a library of DNA sequences comprising nucleic acid sequences encoding heterologous proteins into a low-viscosity mutant filamentous fungus according to the invention, the nucleic acid sequences being operably linked to an expression regulating region. The introduction of the DNA sequences may be carried out in any manner known *per se* for transforming filamentous fungi. Those skilled in the art will appreciate that there are several well-established methods, such as CaCl₂-polyethylene glycol stimulated DNA uptake by fungal protoplasts (Johnstone et al., EMBO J., 1985, 4:1307-1311). A protoplast transformation method is described in the examples. Alternative protoplast or spheroplast transformation methods are known and can be used as have been described in the prior art for other filamentous fungi. Vectors suitable for multicopy integration of heterologous DNA into the funal genome are well-known; see for example Giuseppin et al., WO 91/00920. The use of autonomously replicating plasmids has long been known as an efficient transformation tool for fungi (Gems et al., Gene 1991 98:61-67; Verdoes et al., Gene 1994 146:159-165; Aleksenko and Clutterbuck, Fungal Genetics Biol. 1997 21:373-387; Aleksenko et al., Mol. Gen. Genet. 1996 253:242-246). Details of such methods can be found in many of the cited references, and they are thus incorporated by reference.

Exemplary methods according to the invention, comprising using a low-viscosity mutant strain of *Chrysosporium* or *A. sojae* as starting material for introduction of vectors carrying heterologous DNA, are presented below.

### EXAMPLES

### A. DEVELOPMENT OF COMPACT GROWTH MORPHOLOGY MUTANTS

Various patent applications teach that morphological mutants can be isolated by various ways of screening. WO 96/02653 and WO 97/26330 describe non-defined mutants exhibiting compact morphology. It was found that a proprotein processing mutant of *A*. *sojae* had an unexpected aberrant growth phenotype (hyper-branching) while no detrimental effect on protein production were observed. Culture experiments with this strain revealed a very compact growth phenotype with micropellets. The observed characteristics were not only present in *A. sojae* but other mutated fungi as well, e.g. *A. niger.*

### (1) Construction of an A. niger proprotein processing mutant

To clone the proprotein convertase encoding gene from *A. niger,* PCR was used. Based on the comparison of various proprotein convertase genes from various yeast species and higher eukaryotes, different PCR primers were designed which are degenerated, respectively, 4, 2, 2, 512, 1152, 4608, 2048 and 49152 times. From the amplification using primers PE4 and PE6, two individual clones were obtained of which the encoded protein sequence did show significant homology to the *S. cerevisiae KEX2* sequence. These clones were used for further experiments.

Based on the observed homology to other proprotein convertase genes of the cloned PCR fragment, the corresponding *A. niger* gene was designated *pclA* (from *p*roprotein-*c*onvertase-*l*ike). Southern analysis of genomic digests of *A. niger* revealed that the *pclA* gene was a single copy gene with no closely related genes in the *A. niger* genome, as even at heterologous hybridisation conditions (50 °C; washes at 6xSSC) no additional hybridisation signals were evident. A first screening of an EMBL3 genomic library of *A. niger* N401 (van Hartingsveldt et al., Mol. Gen. Genet. 1987 206:71-75) did not result in any positively hybridising plaques although about 10-20 genome equivalents were screened. In a second screening a full length genomic copy of the *pclA* gene was isolated from an *A. niger* N400 genomic library in EMBL4 (Goosen et al., Curr. Genet. 11:499-503 (1987)).

Of the 8 hybridising plaques which were obtained after screening 5-10 genome equivalents, 6 were still positive after a first rescreening. All these 6 clones most likely carried a full copy of the *pclA* gene, as in all clones (as was observed for the genomic DNA) with the PCR fragment two hybridising EcoRV fragments of 3 and 4 kb were present (the PCR fragment contained an EcoRV restriction site). Based on comparison of the size of other proprotein convertases, together these fragments will contain the complete *pclA* gene with 5' and 3' flanking sequences. The two EcoRV fragments and an overlapping 5 kb EcoRI fragment were subcloned for further characterisation.

Based on the restriction map the complete DNA sequence of the *pclA* gene was determined from the EcoRI and EcoRV subclones. Analysis of the obtained sequence revealed an open reading frame with considerable similarity to that of the *S. cerevisiae KEX2* gene and other proprotein convertases. Based on further comparison two putative intron sequences were identified in the coding region. Subsequent PCR analysis with primers flanking the putative introns, on a pEMBLyex based *A. niger* cDNA library revealed that only the most 5' of these two sequences represented an actual intron. The general structure of the encoded PclA protein was clearly similar to that of other proprotein convertases. The overall similarity of the PclA protein with the other proprotein convertases was about 50%.

To demonstrate that the cloned *pclA* gene is a functional gene encoding a functional protein, the construction of strains devoid of the *pclA* gene was attempted. Therefore, pPCL1A, a *pclA* deletion vector, in which a large part of the *pclA* coding region was replaced for the *A*. *oryzae pyrG* selection marker, was generated. Subsequently, from this vector the 5 kb EcoRI insert fragment was used for transformation of various *A. niger* strains.

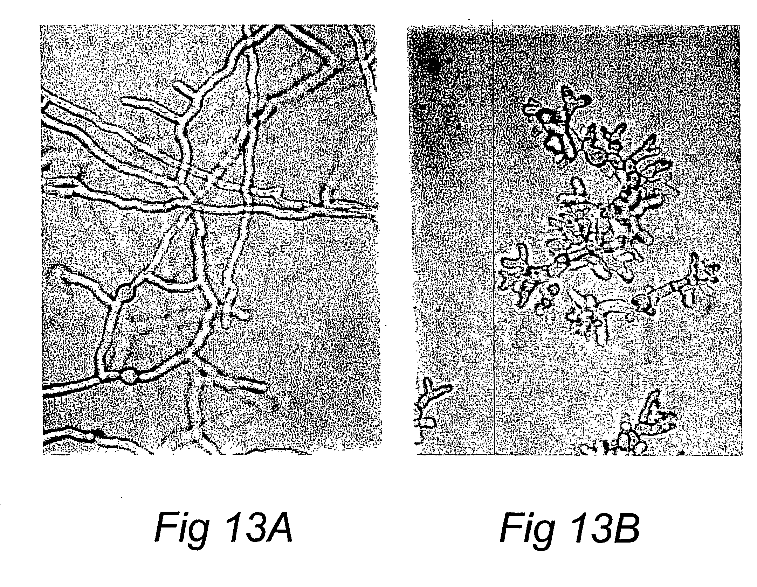

From these transformations (based on *pyrG* selection) numerous transformants were obtained. Interestingly, a fraction of the transformants (varying from 1-50%) displayed a very distinct aberrant phenotype (Figure 13). Southern analysis of several wildtype and aberrant transformants revealed that these aberrant transformants which displayed a severely restricted (compact) growth phenotype, had lost the *pclA* gene. All strains displaying wild-type growth were shown to carry a copy of the replacement fragment integrated adjacent to the wild-type *pclA* gene or at a non-homologous position.

### (2) Construction of an A. sojae proprotein processing mutant

To construct the corresponding mutant in *A. sojae,* functional complementation of the low-viscosity mutant of *A. niger* was carried out by transformation of an *A. niger* pclA mutant with the *A. sojae* ATCC 11906 cosmid library. From the resulting complemented *A. niger* transformants, genomic cosmid clones were isolated, which comprised the *A.sojae* protein processing protease pclA. Partial sequence analysis of the isolated sequences confirmed the cloning of the *A. sojae* pclA gene. Based on the cloned *A. sojae pclA* sequences a gene replacement vector was generated following an approach similar to that described elsewhere in our examples, using the reusable pyrG selection marker described in WO 01/09352.

In addition, a gene disruption vector was constructed carrying the pyrG selection marker and 5' and 3' truncated fragment from the *A. sojae pclA* gene. Both the gene replacement and gene disruption vector were used to generate *pclA* mutants in ATCC 11906 and ATCC 11906 derivatives. Culture experiments with some of the resulting transformants revealed improved morphological characteristics, in particular compact growth morphology and micropellets.. (Figs. 14A and 14B)

### (3) Isolation of alternative A. sojae compact growth mutants

Transformation of *A. sojae* ATCC 11906 and derivatives may be carried out with linear DNA fragments carrying a fungal selection marker. If no specific replicating sequences are provided transformants obtained using this procedure carry the introduced DNA integrated into the genome of the host strain. As the introduced selection marker is from heterologous origin (*A*. *niger*) only heterologous recombination will occur, leading to a collection of transformants carrying the marker DNA at various positions in the genome. This integration is prone to result in disruption of endogenous *A. sojae* sequences, thus resulting in a collection of *A. sojae* mutant strains. This is exemplified by the analysis of a large collection of transformants obtained from *A*. *sojae* ATCC 11906*alpApyrG* using a DNA fragment with the *A. niger* pyrG selection marker. In total several thousand transformants were analysed and from these 5-10 showed a morphologically aberrant phenotype. Amoung these several had a phenotype comparable to the pclA mutants. Similar as described for the cloning of the *A. sojae* pclA gene, the gene corresponding to the mutation could be isolated from the *A. sojae* gene library by complementation of the morphological phenotype. Based on the cloned gene the corresponding gene disruption/deletion mutants can be generated.

### (4) Isolation of Chrysosporium compact growth mutants.

Using a similar PCR based cloning approach as described for the *A. niger* pclA gene a fragment of the *Chrysosporium* proprotein processing gene, termed pcl1, was cloned from a *Chrysosporium* BLUESTAR (TM) gene library. A gene fragment carrying the complete genomic gene copy was subcloned from the pBLUESTAR clone. Based on the obtained subclone a gene disruption vector was generated as described for *A. sojae.* Instead of the pyrG marker, for *Chrysosporium* the repeat flanked version of the *A. niger* pyrE gene was used. Gene disruption-transformation of *Chrysosporium* resulted in strains with a compact growth phenotype.

### B. VISCOSITY DETERMINATIONS

The following operating parameter data ranges have been determined for fungal fermentations using five different fungal organisms. The five fungal organisms compared were strains of *Aspergillus niger, Trichoderma longibrachiatum* 18.2KK (formerly *T. reesei*), *Trichoderma longibrachiatum* X 252, *Chrysosporium lucknowense* strain UV18-25, and *Aspergillus sojae* pclA. Viscosity of a fungal culture varies during the course of a fermentation and varies with nutrient concentration. For the measurements reported here, medium containing between 20 and 100 g/l of a carbohydrate carbon source (*e.g.*, cellulose, lactose, sucrose, xylose, glucose, and the like) is inoculated with the fungus, and the culture allowed to proceed through a "growth phase" during which the carbon source is consumed. Shake flask cultures are shaken at 200 rpm, while one-liter fermentation vessels are stirred with an impeller at 500-1000 rpm. Maximal viscosity typically occurs at or close to the end of the growth phase. At this time the culture is switched to a fed batch mode, wherein a carbon source is fed to the culture at a rate such that the concentration of the carbon source does not rise above about 0.5 g/l. A feed rate of between 1 and 3 g/l/hr is typical.

Viscosity was determined on a Brookfield LVF viscometer using the small sample adapter and spindle number 31, operated at 30 °C. A fresh sample of fermentation broth (10 ml) was placed in the small sample spindle. The spindle speed was adjusted to give a reading in the range 10-80. After four minutes a reading was taken from the viscometer scale. The reading was multiplied by the factor given below to get the viscosity in centipoise (cP).

| Spindle Speed | Multiplication Factor |
|---|---|
| 6 | 50 |
| 12 | 25 |
| 30 | 10 |
| 60 | 5 |

The final viscosity was measured at fermentation end:

| Strain | Final viscosity, cP (mean ± s.d.) |
|---|---|
| *T. longibrachiatum 18.2KK* | (297 ± 173) |
| *A. niger* | 1,500 - 2,000 |
| *T longibrachiatum*_X-252 | ≤ 60 |
| *C. lucknowense* UV18-25 | ≤ 10 |
| *A. sojae* pclA | n.d. |

### C TRANSFORMATION OF CHRYSOSPORIUM, TRICHODERMA AND TOLYPOCLADIUM

Transformation media used were as follows:

| **Mandels Base:** | | **MnP Medium :** |
|---|---|---|
| KH₂PO₄ | 2.0 g/l | Mandels Base with |
| (NH₄)₂SO₄ | 1.4 g/l | Peptone 1 g/l |
| MgSO₄·7H₂O | 0.3 g/l | MES 2 g/l |
| CaCl₂ | 0.3 g/l | Sucrose 100 g/l |
| Oligoelements | 1.0 ml/l | Adjust pH to 5 |

| **MnR** | | **MnP Ca²⁺ :** |
|---|---|---|
| MnP+sucrose | 130 g/l | MnP Medium + |
| Yeast extract | 2.5 g/l | CaCl₂ 2H₂O, 50 mM |
| Glucose | 2.5 g/l | Adjust pH to 6.5 |
| Agar | 15 g/l | |

| | |
|---|---|
| **MnR Soft:** | MnR with only 7.5 g/l of agar. |

| **MPC :** | | |
|---|---|---|
| CaCl₂ | 50 mM | pH 5.8 |
| MOPS | 10 mM | |
| PEG | 40% | |

Media for selection and culture:

| **GS:** | | |
|---|---|---|
| Glucose | 10 g/l | |
| Biosoyase | 5 g/l | [Merieux] |
| Agar | 15 g/l | pH should be 6.8 |

| **PDA:** | |
|---|---|
| Potato Dextrose Agar (Difco) 39 g/l | pH should be 5.5 |

| **MPG:** | |
|---|---|
| Mandels Base with | |
| K Phtalate | 5 g/l |
| Glucose | 30 g/l |
| Yeast extract | 5 g/l |

| **IC1** | |
|---|---|
| 0.5 g/L K2HPO4 | pH 7.0 |
| 0.15 g/L MgSO4·7H20 | |
| 0.05 g/L KCl | |
| 0.007 g/L FeSO4·7H2O | |
| 1 g/L Yeast extract (ohly KAT) | |
| 10 g/L Peptone or Pharmamedia | |
| 10 g/L lactose | |
| 10 g/L glucose | |

The regeneration media (MnR) supplemented with 50 µg/ml phleomycin or 100-150 µg/ml hygromycin is used to select transformants. GS medium, supplemented with 5 µg/ml phleomycin is used to confirm antibiotic resistance.

PDA is a complete medium for fast growth and good sporulation. Liquid media are inoculated with 1/20th of spore suspension (all spores from one 90mm PDA plate in 5 ml 0.1% Tween). Such cultures are grown at 27 °C in shake flasks (200 rpm).

Two untransformed *Chrysosporium* C1 strains and one *Trichoderma reesei* reference strain were tested on two media (GS pH 6.8, and Pridham agar, PA, pH 6.8). To test the antibiotic resistance level spores were collected from 7 day old PDA plates. Selective plates were incubated at 32 °C and scored after 2,4 and 5 days. The C-1 strains NG7C-19 and UV18-25 clearly had a low basal resistance level both to phleomycin and hygromycin, comparable to that for a reference *T. reesei* laboratory strain. This is a clear indication these standard fungal selectable markers can be used in *Chrysosporium* strains. Problems with other standard fungal selectable markers are not expected.

Selection of Sh-ble (phleomycin-resistance) transformed *Chrysosporium* strains was successfully carried out at 50 µg/ml. This was also the selection level used for *T. reesei* thus showing that differential selection can be easily achieved in *Chrysosporium.* The same comments are valid for strains transformed for hygromycin resistance at a level of 150 µg/ml.

The protoplast transformation technique was used on *Chrysosporium* based on the most generally applied fungal transformation technology. All spores from one 90mm PDA plate were recovered in 8ml IC1 and transferred into a shake flask of 50ml IC1 medium for incubation for 15 hours at 35 °C and 200 rpm. After this the culture was centrifuged, the pellet was washed in MnP, brought back into solution in 10ml MnP and 10mg/ml Caylase C₃ and incubated for 30 minutes at 3 5 °C with agitation (150 rpm).

The solution was filtered and the filtrate was subjected to centrifugation for 10 minutes at 3500 rpm. The pellet was washed with 10 ml MnP Ca²⁺. This was centrifuged for 10 minutes at 25 °C. Then 50 microlitres of cold MPC was added. The mixture was kept on ice for 30 minutes whereupon 2.5 ml PMC was added. After 15 minutes at room temperature 500 microlitres of the treated protoplasts were mixed to 3 ml of MnR Soft and immediately plated out on a MnR plate containing phleomycin or hygromycin as selection agent. After incubation for five days at 30 °C transformants were analysed (clones become visible after 48 hours). Transformation efficiency was determined using 10 µg of reference plasmid pAN8-1. The results are presented in the following Table C.

**Table C: Transformation efficiency**

| (using 10 µg of reference plasmid pAN8-1) | | | |
|---|---|---|---|
| | **T. reesei** | **NG7C-19** | **UV18-25** |
| Viability | 10⁶/200 µl | 5 x 10⁶ /200 µl | 5 x 10⁶ / 200 µl |
| Transformants Per 200 µl | 2500 | 10⁴ | 10⁴ |
| Transformants per 10⁶ viable cells | 2500 | 2000 | 2000 |

The results show that the *Chrysosporium* transformant viability is superior to that of *Trichoderma*. The transformability of the strains is comparable and thus the number of transformants obtained in one experiment lies 4 times higher for *Chrysosporium* than for *T. reesei.* Thus the *Chrysosporium* transformation system not only equals the commonly used *T. reesei* system, but even outperforms it. This improvement can prove especially useful for vectors that are less transformation efficient than pAN8-1.

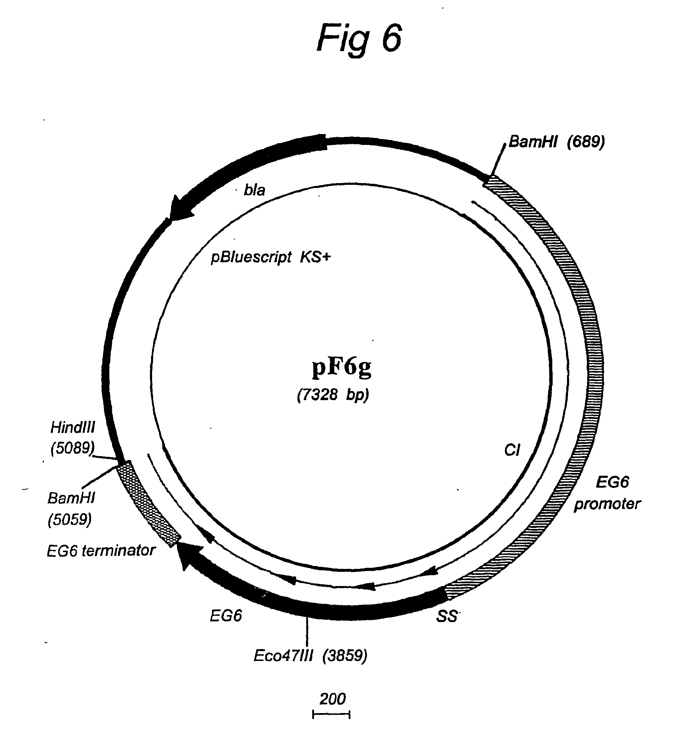

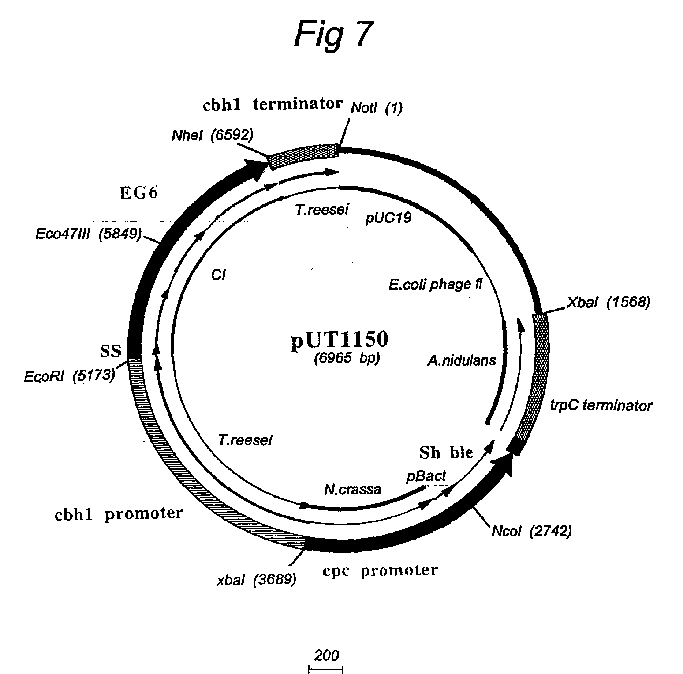

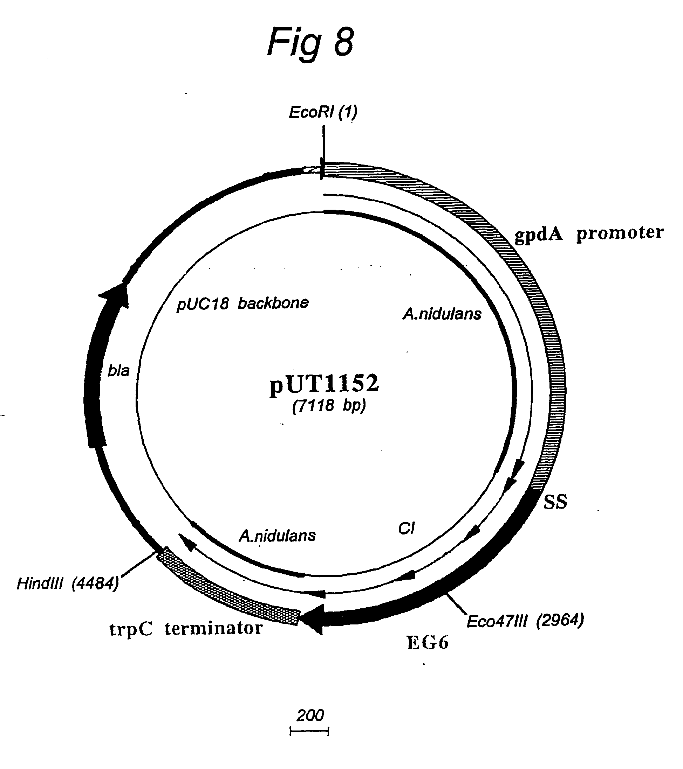

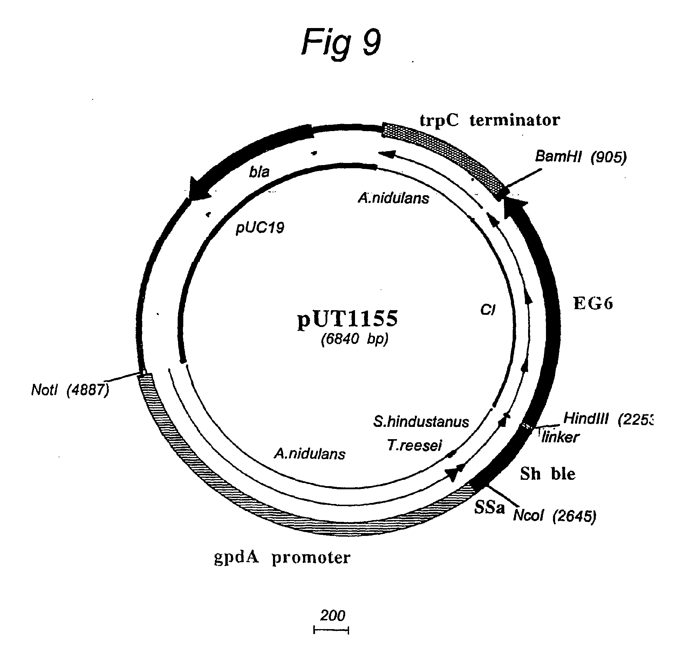

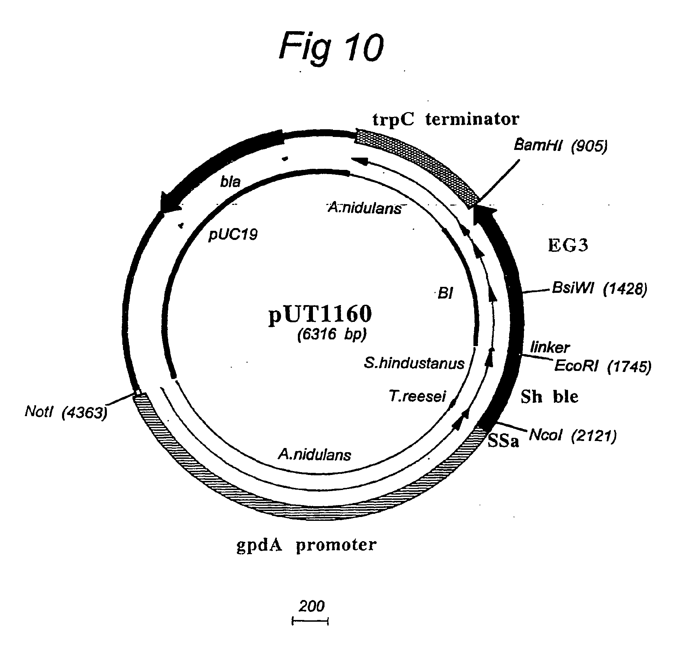

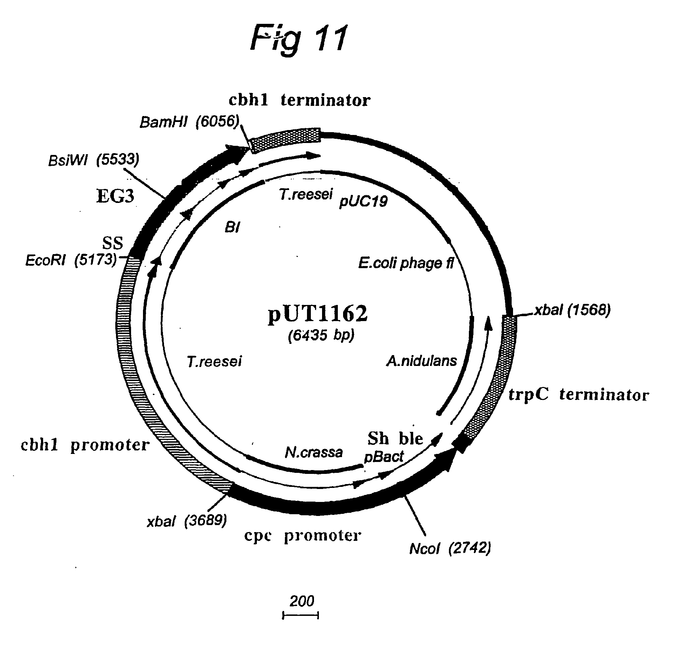

A number of other transformation and expression plasmids were constructed with homologous *Chrysosporium* protein encoding sequences and also with heterologous protein encoding sequences for use in transformation experiments with *Chrysosporium.* The vector maps are provided in Figures 6-11.

The homologous protein to be expressed was selected from the group of cellulases produced by *Chrysosporium* and consisted of endoglucanase 6 which belongs to family 6 (MW 43 kDa) and the heterologous protein was endoglucanase 3 which belongs to family 12 (MW 25 kDa) of *Penicillium.*

pF6g comprises *Chrysosporium* endoglucanase 6 promoter fragment linked to endoglucanase 6 signal sequence in frame with the endoglucanase 6 open reading frame followed by the endoglucanase 6 terminator sequence. Transformant selection is carried out by using cotransformation with a selectable vector.

pUT1150 comprises *Trichoderma reesei* cellobiohydrolase promoter linked to endoglucanase 6 signal sequence in frame with the endoglucanase 6 open reading frame followed by the *T. reesei* cellobiohydrolase terminator sequence. In addition this vector carries a second expression cassette with a selection marker, i.e. the phleomycin resistance gene (Sh-ble gene).

pUT1152 comprises *Aspergillus nidulans* glyceraldehyde-3-phosphate dehydrogenase A promoter linked to endoglucanase 6 signal sequence in frame with the endoglucanase 6 open reading frame followed by the *A. nidulans* anthranilate synthase (trpC) terminator sequence. In addition this vector carries a second expression cassette with a selection marker, i.e. the phleomycin resistance gene (Sh-ble gene).

pUT1155 comprises *A. nidulans* glyceraldehyde-3-phosphate dehydrogenase A promoter linked to *Trichoderma reesei* cellobiohydrolase signal sequence in frame with the carrier protein Sh-ble which in turn is linked in frame to the endoglucanase 6 open reading frame followed by the *A. nidulans* trpC terminator sequence. This vector uses the technology of the carrier protein fused to the protein of interest which is known to very much improve the secretion of the protein of interest.

pUT1160 comprises *Aspergillus nidulans* glyceraldehyde-3-phosphate dehydrogenase A promoter linked to *Trichoderma reesei* cellobiohydrolase signal sequence in frame with the carrier protein Sh-ble which in turn is linked in frame to the endoglucanase 3 open reading frame of Penicillium followed by the *A. nidulans* trpC terminator sequence.

pUT1162 comprises *Trichoderma reesei* cellobiohydrolase promoter linked to endoglucanase 3 signal sequence in frame with the endoglucanase 3 open reading frame of *Penicillium* followed by the *T. reesei* cellobiohydrolase terminator sequence. In addition this vector carries a second expression cassette with the phleomycin resistance gene (Sh-ble gene) asa selection marker.

It will be apparent to those skilled in the art that a sample of genomic or cDNA can be readily sheared or digested into protein-encoding fragments, and the fragments ligated into vectors such as those illustrated herein so as to produce a library of expression vectors. It will be further apparent that methods employing co-transfection are applicable, and that autonomously replicating vectors or integrating vectors may be employed to transfect filamentous fungi with such a library of vectors.

**Table D: Comparative transformations**

| Vector | Strain | Transformation | No of transf. |
|---|---|---|---|
| pUT1150 | UV18-25 | selection on phleomycin | 285 |
| | *T. geodes* | selection on phleomycin | 144 |
| | | | |
| pUT1152 | UV18-25 | cotransformation pAN8.1 | 398 |
| | *T. geodes* | cotransformation pAN8.1 | 45 |
| | | | |
| pF6g | UV18-25 | cotransformation pAN8.1 | 252 |
| | *T. geodes* | cotransformation pAN8.1 | 127 |
| | | | |
| pUT1162 | UV18-25 | selection on phleomycin | >400 |
| | *T. geodes* | (n.d.) | |

Table D shows the results of transformation of both *Chrysosporium* UV18-25 and *Tolypocladium geodes.* The transformation protocol used is described below in the section for heterologous transformation.

### D. HETEROLOGOUS AND HOMOLOGOUS EXPRESSION IN CHRYSOSPORIUM TRANSFORMANTS

C1 strains (NG7C-19 and/or UV18-25) were tested for their ability to secrete various heterologous proteins: a bacterial protein (*Streptoalloteichus hindustanus* phleomycin-resistance protein, Sh-ble), a fungal protein (*Trichoderma reesei* xylanase II, XYN2) and a human protein (the human lysozyme, HLZ). The details of the process are as follows:

### (1) C1 secretion of Streptoalloteichus hindustanus phleomycin-resistance protein (Sh-ble).

C1 strains NG7C-19 and UV18-25 were transformed by the plasmid pUT720 (ref. 1). This vector presents the following fungal expression cassette:
- *Aspergillus nidulans* glyceraldehyde-3-phosphate dehydrogenase (gpdA) promoter (ref 2)
- A synthetic *Trichoderma reesei* cellobiohydrolase I (cbh1) signal sequence (refs 1, 3)
- *Streptoalloteichus hindustanus* phleomycin-resistance gene Sh-ble (ref. 4)
- *Aspergillus nidulans* tryptophan-synthase (*trpC*) terminator (ref. 5)

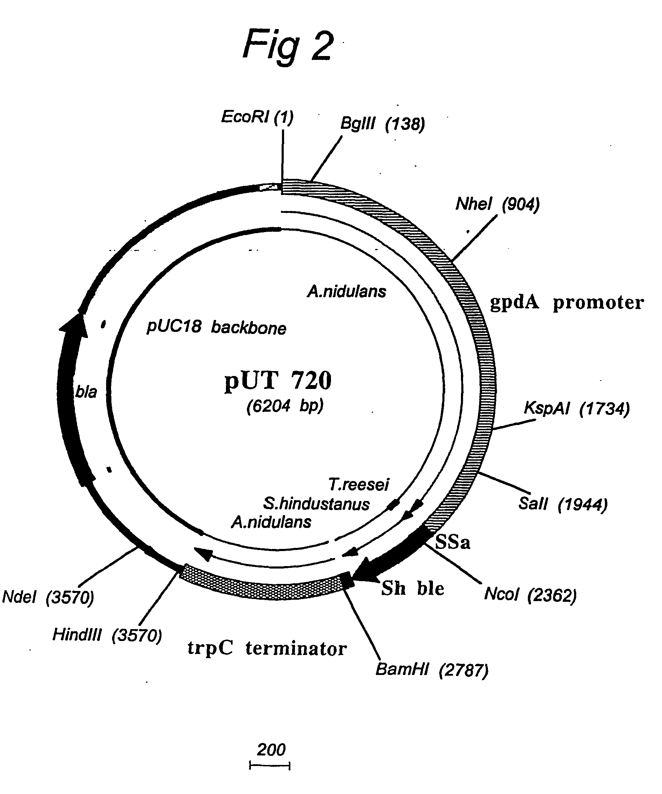

The vector also carries the beta-lactamase gene (*bla*) and *E. coli* replication origin from plasmid pUC18 (Ref 6). The detailed plasmid map is provided in Figure 2.

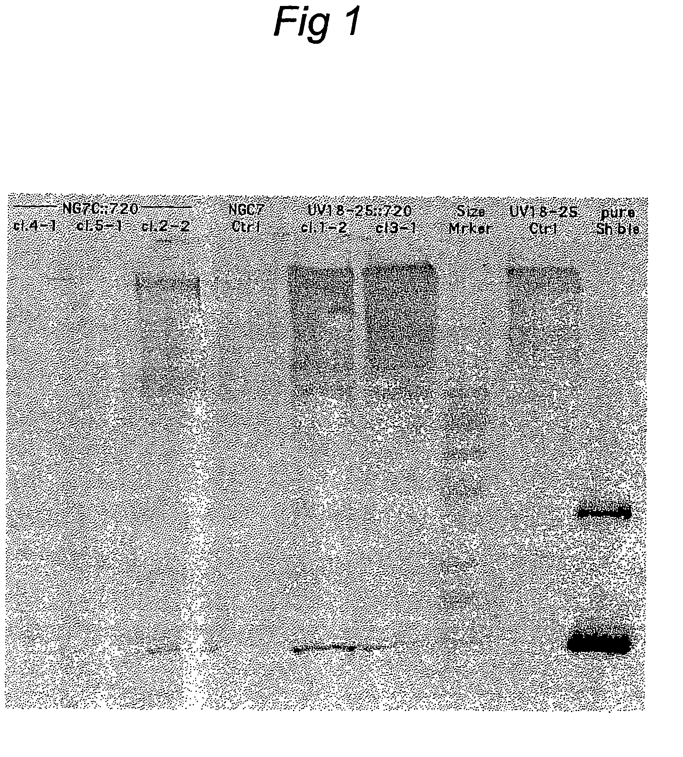

C1 protoplasts were transformed according to Durand *et al.* (ref. 7) adapted to C1: All spores from one 90mm PDA plate of untransformed C1 strain were recovered in 8ml IC1 and transferred into a shake flask with 50ml IC1 medium for incubation 15 hours at 35 °C and 150 rpm. Thereupon, the culture was spun down, the pellet washed in MnP, resolved in 10ml MnP + 10mg/ml Caylase C₃, and incubated 30 min at 35 °C with agitation (150 rpm). The solution was filtered and the filtrate was centrifuged 10 min at 3500 rpm. The pellet was washed with 10ml MnPCa²⁺. This was spun down 10min at 3500 rpm and the pellet was taken up into 1ml MnPCa²⁺. 10µg of pUT720 DNA were added to 200µl of protoplast solution and incubated 10min at room temperature (ca. 20 °C). Then, 50µl of cold MPC was added. The mixture was kept on ice for 30min whereupon 2.5ml PMC was added. After 15min at room temperature 500µl of the treated protoplasts were mixed to 3ml of MnR Soft and immediately plated out on a MnR plate containing phleomycin (50µg/ml at pH6.5) as selection agent. After 5 days incubation at 30 °C, transformants were analysed (clones start to be visible after 48 hours). The Sh-ble production of C1 transformants (phleomycin-resistant clones) was analysed as follows: Primary transformants were toothpicked to GS+phleomycin (5µg/ml) plates and grown for 5 days at 32 °C for resistance verification. Each validated resistant clone was subcloned onto GS plates. Two subclones per transformant were used to inoculate PDA plates in order to get spores for liquid culture initiation. The liquid cultures in IC1 were grown 5 days at 27 °C (shaking 200 rpm). Then, the cultures were centrifuged (5000g, 10min.) and 500µl of supernatant were collected. From these samples, the proteins were precipitated with TCA and resuspended in Western Sample Buffer to 4 mg/ml of total proteins (Lowry method, Ref. 8). 10µl (about 40µg of total proteins) were loaded on a 12% acrylamide/SDS gel and run (Mini Trans-Blot™ system, BioRad Laboratories). Western blotting was conducted according to BioRad instructions (Schleicher & Schull 0.2µm membrane) using rabbit anti-Sh-ble antiserum (Societe Cayla, Tolouse FR, Catalog #ANTI-0010) as primary antibody. The results are shown in Figure 1 and Table E.

**Table E: Sh-ble estimated production levels in C1**

| | Estimated Sh-ble quantity on the Western blot | Estimated Sh-ble concentration in the production media |
|---|---|---|
| Untransformed NG7C-19 | Not detectable | |
| NG7C-19::720 clone 4-1 | 25 ng | 0.25 mg/l |
| NG7C-19::720 clone 5-1 | 25 ng | 0.25 mg/l |
| NG7C-19::720 clone 2-2 | 250 ng | 2.5 mg/l |
| Untransformed UV18-25 | Not detectable | |
| UV18-25::720 clone 1-2 | 500 ng | 5.0 mg/l |
| UV18-25::720 clone 3-1 | 250 ng | 2.5 mg/l |

These data show that:
1) The heterologous transcription/translation signals from pUT720 are functional in *Chrysosporium.*
2) The heterologous signal sequence of pUT720 is functional in *Chrysosporium.*
*3) Chrysosporium* can be used a host for the secretion of heterologous bacterial proteins.

### (2) C1 secretion of human lysozyme (HLZ).

C1 strains NG7C-19 and UV18-25 were transformed by the plasmid pUT970G (ref. 9). This vector presents the following fungal expression cassette:
- *Aspergillus nidulans* glyceraldehyde-3-phosphate dehydrogenase (*gpdA*) promoter (ref. 2)
- A synthetic *Trichoderma reesei* cellobiohydrolase I *(cbh1)* signal sequence (refs. 1, 3)
- *Streptoalloteichus hindustanus* phleomycin-resistance gene Sh-ble 4 used as carrier protein (ref 10)
- *Aspergillus niger* glucoamylase *(glaA2)* hinge domain cloned from plasmid pAN56-2 (refs. 11, 12)
- A linker peptide (LGERK) featuring a KEX2-like protease cleavage site (ref. 1)
- A synthetic human lysozyme gene (*h*/*z*) (ref. 10)
- *Aspergillus nidulans* tryptophan-synthase (*trpC*) terminator (ref. 5)

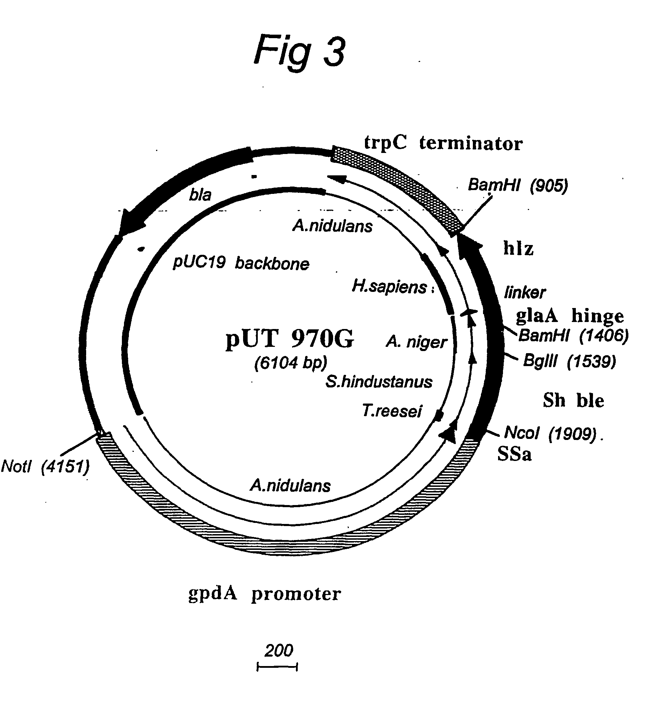

The vector also carries the beta-lactamase gene (*bla*) and *E*. *coli* replication origin from plasmid pUC18 6. The detailed plasmid map is provided in Figure 3.

C1 protoplasts were transformed with plasmid pUT970G following the same procedure already described in example 1. The fusion protein (Sh-ble :: GAM hinge :: HLZ) is functional with respect to the phleomycin-resistance thus allowing easy selection of the C1 transformants. Moreover, the level ofphleomycin resistance correlates roughly with the level of hlz expression.

The HLZ production of C1 transformants (phleomycin-resistant clones) was analysed by lysozyme-activity assay as follow: Primary transformants were toothpicked to GS+phleomycin (5µg/ml) plates (resistance verification) and also on LYSO plates (HLZ activity detection by clearing zone visualisation (refs. 1, 10). Plates were grown for 5 days at 32 °C. Each validated clone was subcloned onto LYSO plates. Two subclones per transformant were used to inoculate PDA plates in order to get spores for liquid culture initiation. The liquid cultures in IC1 were grown 5 days at 27 °C (shaking 180 rpm). Then, the cultures were centrifuged (5000g, 10min.). From these samples, lysozyme activity was measured according to Mörsky *et al.* (ref 13)

**Table F: Active HLZ production levels in C1**

| | Active HLZ concentration in culture media |
|---|---|
| Untransformed NG7C-19 | 0 mg/l |
| NG7C-19::970G clone 4 | 4 mg/l |
| NG7C-19::970G clone 5 | 11 mg/l |
| Untransformed UV 18-25 | 0 mg/l |
| UV18-25::970G clone 1 | 8 mg/l |
| UV18-25::970G clone 2 | 4 mg/l |
| UV18-25::970G clone 3 | 2 mg/l |
| UV18-25::970G clone 2 | 2.5 mg/l |

These data show that:
1) Points 1 & 2 from example 1 are confirmed.
2) Sh-ble is functional in *Chrysosporium* as resistance marker.
3) Sh-ble is functional in *Chrysosporium* as carrier protein.
4) The KEX2-like protease cleavage site is functional in *Chrysosporium* (otherwise HLZ would not be active).
*5) Chrysosporium* can be used as host for the secretion of heterologous mammalian proteins.

### (3) C1 secretion of Trichoderma reesei xylanase II (XYN2).

C1 strain UV18-25 was transformed by the plasmids pUT1064 and pUT1065. pUT1064 presents the two following fungal expression cassettes:

The first cassette allows the selection of phleomycin-resistant transformants:
- *Neurospora crassa* cross-pathway control gene 1 (*cpc-1*) promoter (ref. 14)
- *Streptoalloteichus hindustanus* phleomycin-resistance gene *Sh-ble* (ref. 4)
- *Aspergillus nidulans* tryptophan-synthase (*trpC*) terminator (ref. 5)

The second cassette is the xylanase production cassette:
- *T. reesei* strain TR2 *cbh1* promoter (ref. 15)
- *T. reesei* strain TR2 *xyn2* gene (including its signal sequence) (ref. 16)
- *T. reesei* strain TR2 *cbh1* terminator (ref. 15)

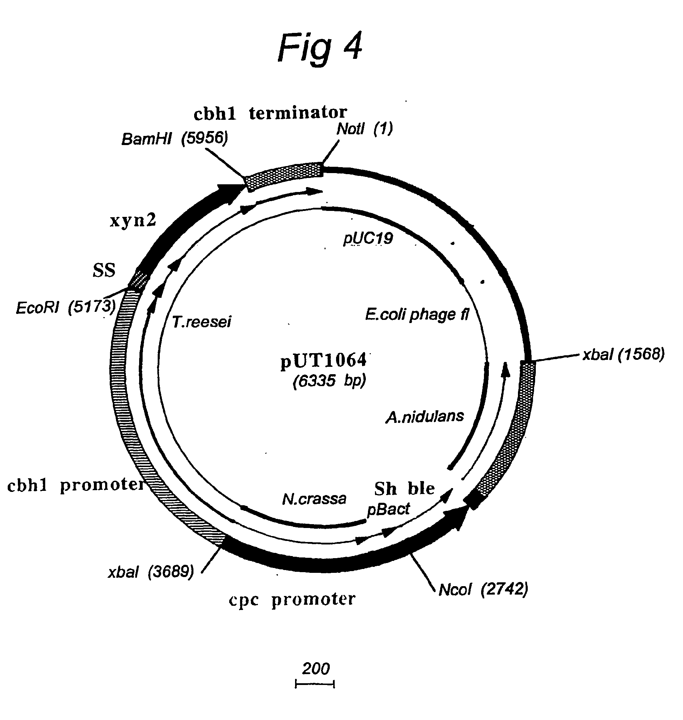

The vector also carries an *E*. *coli* replication origin from plasmid pUC19 (ref. 6). The plasmid detailed map is provided in figure 4.
pUT1065 presents the following fungal expression cassette:
- *A. nidulans* glyceraldehyde-3-phosphate dehydrogenase (*gpdA*) promoter (ref. 2)
- A synthetic *T. reesei* cellobiohydrolase I (*cbh1*) signal sequence (refs. 1, 3) *- S. hindustanus* phleomycin-resistance gene *Sh-ble* 4 used as carrier-protein (ref. 10)
- A linker peptide (SGERK) featuring a KEX2-like protease cleavage site (ref. 1)
- *T. reesei* strain TR2*xyn2* gene (without signal sequence) (ref. 16)
- *A. nidulans* tryptophan-synthase (*trpC*) terminator (ref. 5)

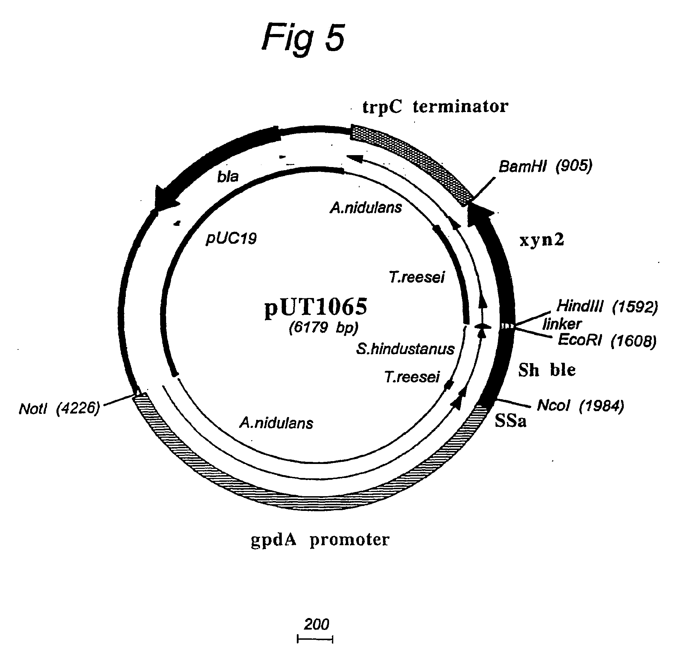

The vector also carries the beta-lactamase gene (*bla*) and an *E. coli* replication origin from plasmid pUC18 (Ref. 6). The plasmid detailed map is provided in Figure 5.

C1 protoplasts were transformed with plasmid pUT1064 or pUT1065 following the same procedure already described in example 1. The fusion protein in plasmid pUT1065 (Sh-ble :: XYN2) is functional with respect to the phleomycin-resistance thus allowing easy selection of the C1 transformants. Moreover, the level of phleomycin resistance correlates roughly with the level of *xyn2* expression. In pUT1064, *xyn2* was cloned with its own signal sequence.

The xylanase production of C1 transformants (phleomycin-resistant clones) was analysed by xylanase-activity assay as follow: Primary transformants were toothpicked to GS+phleomycin (5µg/ml) plates (resistance verification) and also on XYLAN plates (Ref. 17), where xylanase activity is detected by observation of a clearing zone. Plates were grown for 5 days at 32 °C. Each validated clone was subcloned onto XYLAN plates. Two subclones per transformant were used to inoculate PDA plates in order to get spores for liquid culture inoculation. The liquid cultures in IC1+ 5g/l K⁺ Phtalate were grown 5 days at 27 °C (shaking 180 rpm). Then, the cultures were centrifuged (5000g, 10 min.). From these samples, xylanase activity was measured by DNS Technique according to Miller *et al.* (ref 18)

**Table G: Active XYN2 production levels in C1 (best producers)**

| | Active xylanase II concentration in culture media | Xylanase II specific activityin culture media |
|---|---|---|
| Untransformed UV18-25 | 3.9 U./ml | 3.8 U./mg total prot. |
| UV18-25::1064 clone 7-1 | 4.7 U./ml | 4.7 U./mg total prot. |
| UV18-25::1064 clone 7-2 | 4.4 U./ml | 4.3 U./mg total prot. |
| UV18-25::1065 clone 1-1 | 29.7 U./ml | 25.6 U./mg total prot. |
| UV18-25::1065 clone 1-2 | 30.8 U./ml | 39.4U./mg total prot. |

These data show that:
1) Points 1 to 4 from example 2 are confirmed.
2) C1 can be used as host for the secretion of heterologous fungal proteins.

### (4) Summary

Table H shows the results for the plasmids with which transformation of UV18-25 was carried out. The Table shows expression levels for endoglucanase and cellobiohydrolase using heterologous expression regulating sequences and signal sequences and also with homologous expression regulating sequences and signal sequences. The details of the various plasmids can be derived elsewhere in the description and from the figures. The production occurs at alkaline pH at a temperature of 35 °C.

**Table H: Expression data of transformed UV18-25 strain**

| **(% relative to parent UV18-25 strain)** | | | | | | |
|---|---|---|---|---|---|---|
| Culture | Total proteins mg/ml | CMCase | | β-glucanase | | pH value |
| | | u/ml | u/mg | u/ml | u/mg | |
| UV18-25 | 100% | 100% | 100% | 100% | 100% | 7.90 |
| 1150-23 | 94% | 105% | 111% | 140% | 149% | 7.90 |
| -30 | 96% | 105% | 110% | 145% | 151% | 8.10 |
| 1152-3 | 94% | 112% | 120% | 147% | 156% | 7.85 |
| -4 | 100% | 105% | 105% | 132% | 132% | 7.90 |
| 1160-2 | 69% | 81% | 118% | 90% | 131% | 7.90 |
| -4 | 73% | 72% | 98% | 83% | 114% | 8.35 |
| -1 | 92% | 95% | 103% | 120% | 130% | 8.45 |
| 1162-1 | 102% | 105% | 103% | 145% | 142% | 8.20 |
| -11 | 112% | 109% | 98% | 115% | 103% | 8.20 |
| F6g-20 | 104% | 102% | 98% | 130% | 125% | 7.90 |
| -25 | - | - | - | - | - | - |

| | | | | | | |
|---|---|---|---|---|---|---|
| Culture conditions (shake flask): 88h, 35 °C, 230 rpm | | | | | | |

### E. CONSTRUCTION OF AN ASPERGILLUS SOJAE GENE LIBRARY

### (1) Vector library

Genomic DNA of *A. sojae* was isolated from protoplasts obtained from ATCC 11906 using a previously described protocol (Punt, van den Hondel, Methods Enzymol. 1992 216:447-457). After isolation DNA was extracted from the protoplasts using the protocol described by Kolar et al., Gene 1988 62:127-34. Subsequently the DNA was partially digested with MboI to result in DNA fragments of an average size of 30-50 kb.

Vector pAOpyrGcosarp1, which was used for the construction of the gene library was constructed by ligation of a 3 kb BamHI-HindII fragment from pANsCos1 (Osiewacz, Curr Genet. 1994 26:87-90) and a 3.2 kb Acc65I-HindIII fragment from pAO4.2 (De Ruiter-Jacobs et al., Curr. Genet. 1989 16:159-63) in Acc65I-BamHI digested pHELP1 (Gems et al., Gene 1991 98:61-67). This cosmid vector carries the *A. oryzae* pyrG selection marker and is self-replicating in filamentous fungi.

MboI digested genomic DNA was ligated to BamHI-digested pAOpyrGcosarp1, and the ligation mixture was packaged into phage particles using the Stratagene Supercos1 vector kit (Stratagene Inc., La Jolla CA). This resulted in a total of *ca.* 30,000 individual clones, representing an approximate 30-fold representation of the *A. sojae* genome. Stocks (in 15% glycerol) of pools of the resulting clones were stored at -80°C for later use.

### (2) High-frequency transformation

An *A. sojae* ATCC 11906 pyrG mutant was selected as a fluoroorotic acid-resistant derivative from ATCC 11906, as described in WO 01/09352. This strain, *A. sojae* ATCC 11906pyrG, was transformed with two vectors carrying the *A. niger* pyrG gene. One vector pAB4-1 (van Hartingsveldt et al., Mol. Gen. Genet. 206:71-75 (1987)) carries only the pyrG gene, whereas pAB4-arp1 (Verdoes et al., Gene 146:159-165 (1994)) carries the pyrG gene and the *A. nidulans* AMA1 sequence. Transformation of ATCC 11906pyrG results in 5-10 transformants per microgram DNA from pAB4-1, whereas with pAB4-arp1 frequency were at least 10-100 fold higher. Phenotypic analysis of the transformants revealed that the pyrG phenotype of the pAB4-arp1 transformants was maintained only under continuous selection, whereas the pAB4-1 transformants were stable with and without selection for the pyrG phenotype. These results confirm autonomous replication of the introduced plasmid DNA in pAB4-arp1 transformants. Similar results were obtained with alternative fungal transformation vectors carrying the AMA1 sequence or derivatives thereof., e.g. pAOpyrGcosarp1.

### (3) Construction of a fungal transformant library

*A. sojae* ATCC 11906pyrG or relevant mutants, in particular compact morphology mutants thereof, was transformed with an *A. sojae* gene library based on transformation vector pAOpyrGcosarp1. This vector results in a high frequency of transformants with freely replicating vector copies. Fungal protoplasts were treated as described in Punt and van den Hondel, Methods Enzymol. 1992 216:447-457 with DNA from a cosmid library carrying genomic fungal DNA clones from *A. sojae* or *Chrysosporium* and serial dilutions of the transformed protoplasts were plated on selective agar plates to determine the transformation frequency obtained. The remaining protoplasts were regenerated in selective medium for a few hours and stored at 4°C. Based on the results obtained for the transformation frequency (which depending of the experiment will reach values up to several thousand transformants per microgram of cosmid library DNA), limiting dilutions of the regenerated protoplasts were plated in microtiter plates of 96, 248, or alternative well format, resulting in one transformed protoplast per well. Plates were incubated at 35°C to form fungal biomass. The resulting transformant library is used for further experiments.

A similar strategy was used for the construction of a collection of fungal transformants carrying mutant alleles of *Chrysosporium* CBH1. This strategy can also be used with a library of mutants derived from any other gene of interest, whether generated by mutagenesis, gene shuffling or gene-evolution approaches.

### F. INDUCTION OF SPORULATION IN SUBMERGED FERMENTATION

Many fungi, such as *Aspergillus sojae,* do not show sporulation under submerged fermentation. Here we describe a previously unknown approach to obtain sporulation under these conditions. *A. sojae* ATCC 11906 and in particular compact growth morphology mutants thereof were grown in a synthetic growth medium supplemented with Yeast extract. Under these conditions rapid accumulation of biomass occurs in both static and agitated cultures. However, no sporulation occurs in the culture fluid. A similar growth medium with the addition of 0.6 g/kg EDTA results in considerable yields of spores reaching up to 10⁹ spores per ml culture fluid after incubation of 2-4 days at 35 °C

### SYNTHETIC MEDIUM (+/- EDTA):

| | g/kg medium |
|---|---|
| KH₂PO₄ | 2.5 |
| NH₄Cl | 7.2 |
| MgSO₄·7H₂0 | 0.7 |
| CaCl₂·2H₂0 | 0.2 |
| Yeast Extract | 20 |
| ZnSO₄·7H₂0 | 0.015 |
| CoCl₂·6H₂0 | 0.005 |
| CuSO₄·5H₂0 | 0.016 |
| FeSO₄·7H₂0 | 0.040 |
| H₃BO₄ | 0.005 |
| KI | 0.003 |
| MnCl₂·2H20 | 0.012 |
| Na₂MoO₄·2H20 | 0.003 |
| EDTA | (0.6 or 0.0) |
| PH adjusted to 5.5 with | NaOH/H₃PO₄ |

### G. TRANSFORMATION SYSTEMS FOR CHRYSOSPORIUM AND ASPERGILLUS

### (1) Cloning of the A. niger orotate p-ribosyl transferase gene pyrE

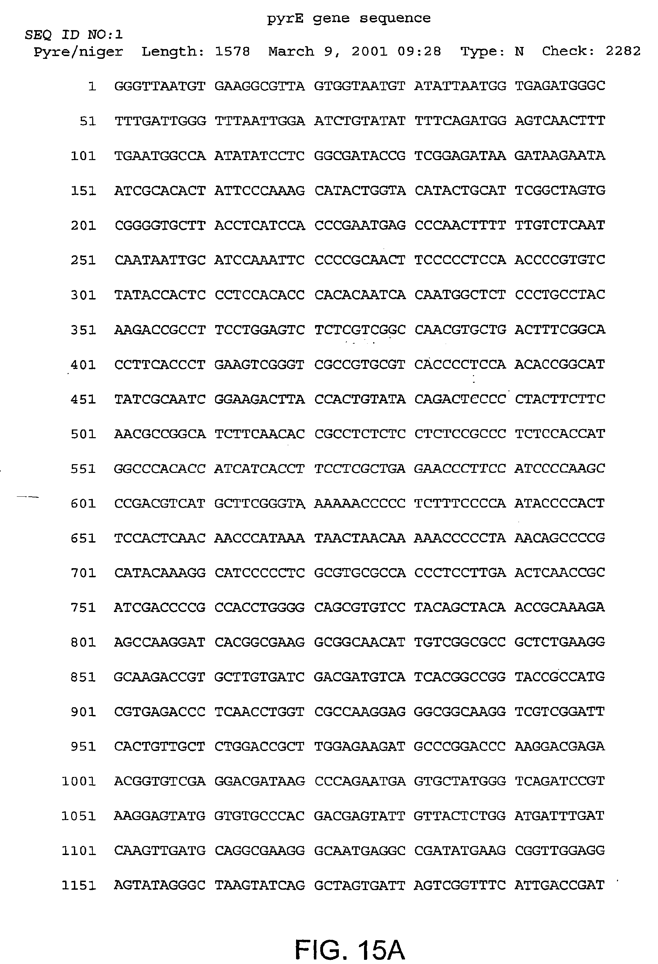

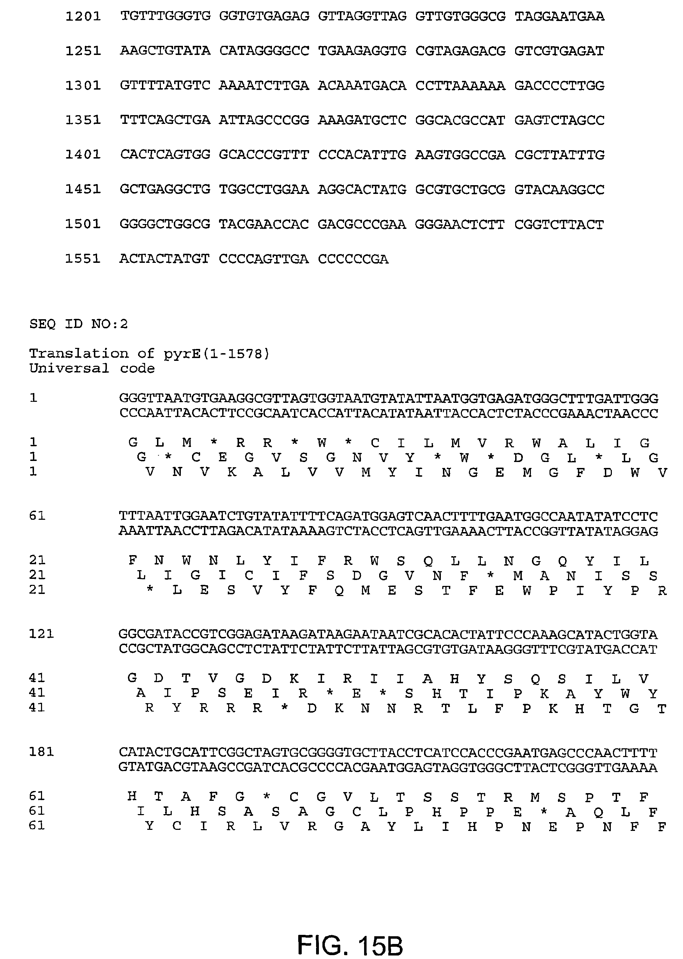

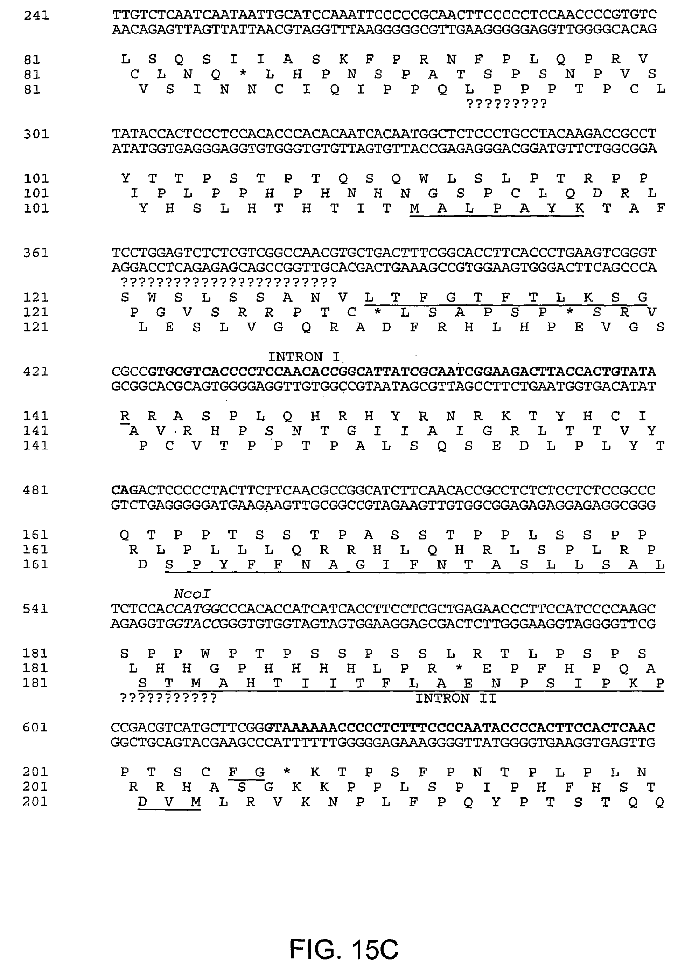

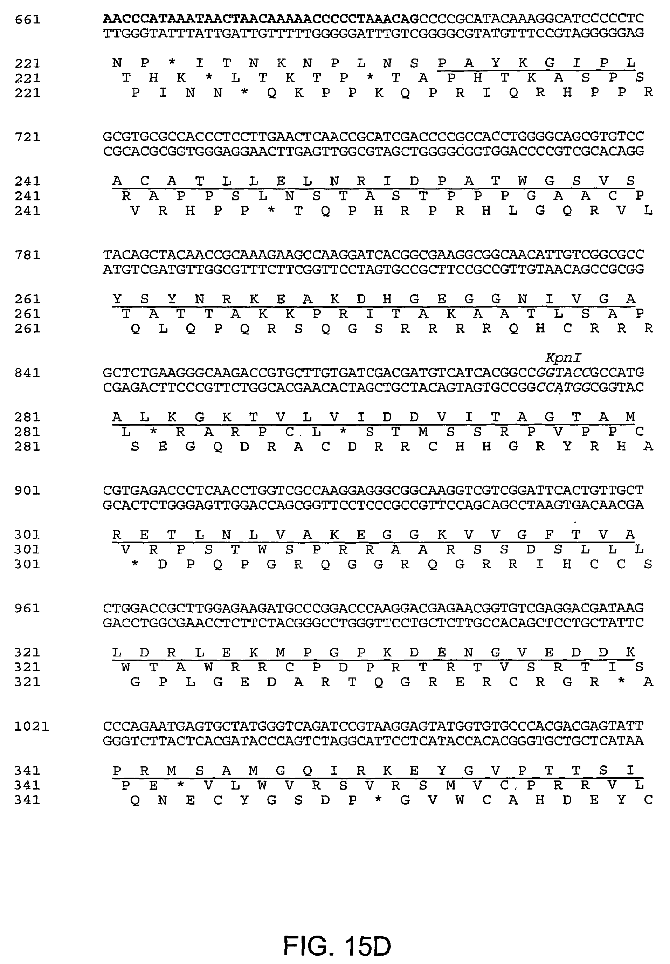

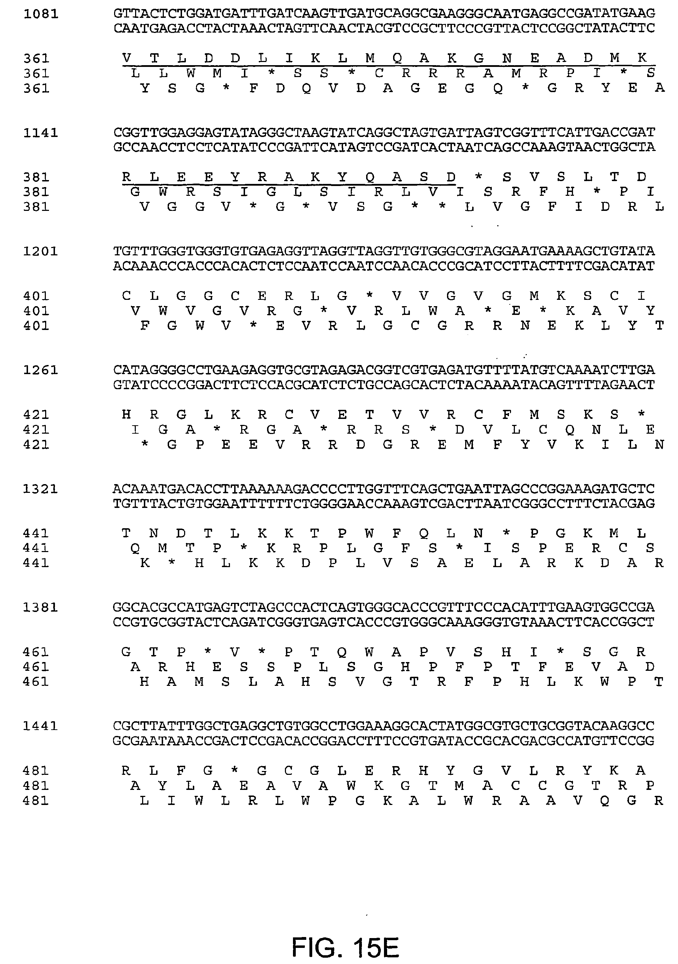

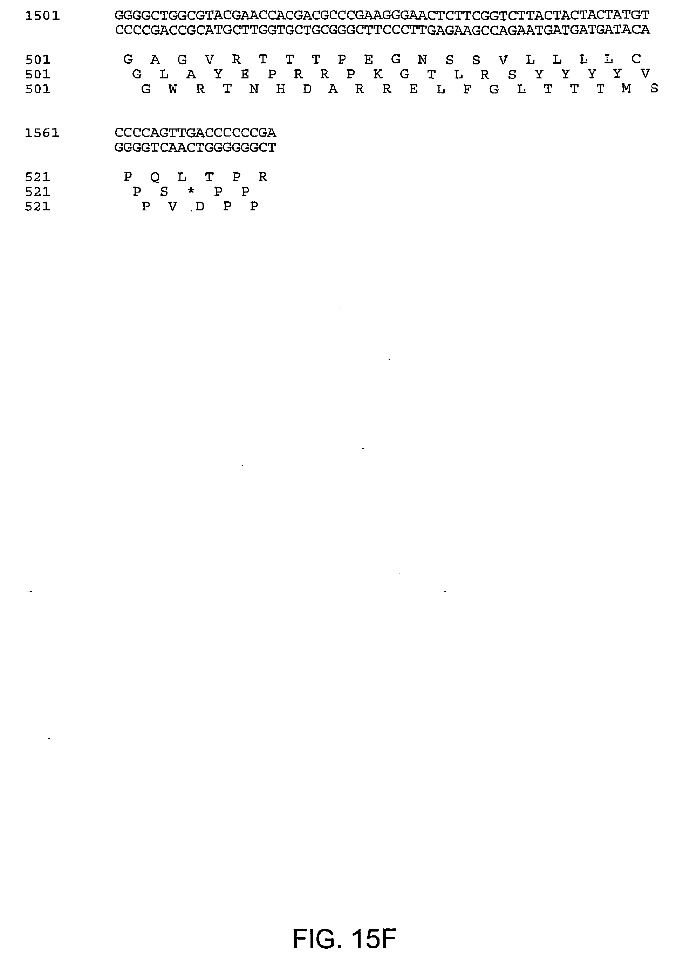

Numerous versatile transformation systems for filamentous fungi are based on the use of uridine-requiring mutant strains. These mutant strains are either deficient in orotidine 5 phosphate decarboxylase (OMPD) or orotate p-ribosyl transferase (OPRT). (T. Goosen et al., Curr Genet. 1987,11:499-503; J. Begueret et al., Gene. 1984 32:487-92.) Previously we have isolated the *A. niger* OMPD gene pyrG (W. van Hartingsveldt et al., Mol. Gen. Genet. 1987 206:71-5). The cloning of the *A. niger* OPRT gene (pyrE) was carried out by complementation of an *A. niger* FOA-resistant uridine-requiring non-pyrG mutant. For complementation an *A. niger* cosmid library in vector pAOpyrGcosarp1 was used. From the complementing transformants, genomic cosmid clones were isolated, carrying the complementing *A. niger* gene, termed now pyrE. A 5.5 kb SstII fragment carrying the pyrE gene was cloned in pBLUESCRIPT (TM) (Stratagene) resulting in vector pBLUEpyrE. A 1.6 kb fragment of this vector spanning the pyrE coding region was sequenced to confirm the location of the OPRT gene (See Fig. 15).

### (2) Auxotrophic transformation system for Chrysosporium lucknowense

Uridine-requiring *Chrysosporiun luknowense* strains were selected as fluoroorotic acid resistant derivatives from C1 and UV18-25 by methods described in PCT publication WO 01/09352. Selection of fluoro-orotic acid resistant derivatives may result in the isolation of two types of uridine-requiring mutants, i.e. either orotidine 5 phosphate decarboxylase (OMPD) mutants or orotate p-ribosyl transferase (OPRT)mutants (T. Goosen et al., Curr Genet. 1987, 11:499-503). To determine the nature of the *Chrysosporium* mutants obtained, transformation experiments were carried out with the available *A. niger* genes pyrG (OMPD; vector pAB4-1, W. van Hartingsveldt et al., Mol. Gen. Genet. 1987 206:71-5) and pyrE (pBLUE-pyrE; OPRT). As shown in Table I, only transformation of the mutant strains with the pyrE gene resulted in prototrophic transformants, implying that the *Chrysosporium* strains are OPRT mutants. Following the *Chrysosporium* gene nomenclature we have adopted, the mutants were designated *pyr5.*

**Table I**

| **Gene** | **Source** | **Vector¹⁻⁴** | **UV18FOA^{R}#4** | **C1#B** |
|---|---|---|---|---|
| OMPD *(PyrG*/*pyr4)* | *Aspergillus niger* | *pAB4-1* | - | - |
| | *Aspergillus oryzae* | *pA04-2* | - | - |
| | *Neurospora crassa* | *pDJB3* | - | - |
| *OPRT (pyrE*/*pyr5)* | *Aspergillus niger* | *pBLUEpyrE* | + | + |
| | | | | |

| | | | | |
|---|---|---|---|---|
| 1. pAB4-1: W. van Hartingsveldt et al., Mol. Gen. Genet. 1987 206:71-5. 2. pAO4-2: Y. de Ruiter-Jacobs et al., Curr. Genet. 1989 16:159-63. 3. pDJB3: D. Ballance, G. Turner, Mol. Gen. Genet. 1986 202:271-5. 4. pBLUEpyrE: *vide supra* | | | | |

### (3) Construction and use of autonomously replicating fungal transformation vectors.

Based on vector pBLUEpyrE two derivatives were generated carrying sequences providing autonomous replicative characteristics to the vectors when introduced in filamentous fungi. A 5.5 kb HindIII fragment carrying the *Aspergillus nidulans* AMA1 sequences (J: Verdoes et al., Gene 1994 146:159-65) was introduced in the unique HindIII site of pBLUEpyrE resulting in pBLUEpyrE-AMA. A 2.1 kb (partial) HindIII fragment carrying human telomeric sequences (A. Aleksenko, L. Ivanova, Mol. Gen. Genet. 1998 260:159-64) was introduced in the unique HindIII site of pBLUEpyrE resulting in pBLUEpyrE-TEL. These vectors were introduced into *Aspergillus* and *Chrysosporium* OPRT mutant strains resulting in prototrophic transformants. Several of the obtained transformants showed the ragged phenotype characteristic of transformants carrying freely replicating plasmids (J. Verdoes et al., Gene 1994 146:159-65).

### (4) Transformation of Chrysosporium lucknowense

The protocol is based on a procedure originally used for *Aspergillus* transformation (P. Punt, C. van den Hondel, Methods in Enzymology 1992 216:447-457). Rich medium (250 ml) was inoculated with 10⁶ spores/ml of the pyr5 *Chrysosporium* mutant (*supra*) in a 1L Erlenmeyer flask. The culture was grown for 24-48 hours at 35 °C in an air incubator (300 rpm). The mycelium was filtered through a sterile Miracloth(TM) filter (Calbiochem) and washed with ca. 100 ml 1700 mosmol NaCl/CaCl₂ (0.27 M CaCl₂/ 0.6 M NaCl). The mycelium was weighed and then kept on ice. Caylase(TM) (Cayla) was added (20 mg per gram mycelium) and 1700 mosmol NaCl/CaCl₂ (3.3ml/g mycelium) and the suspension was incubated in a 33 °C air incubator (100 rpm). The protoplasting was followed under the microscope. After 1-3 hours of incubation, most of the mycelium was digested, leaving mostly protoplasts in the microscopic view of the preparation. The protoplasts were filtered through a sterile Myracloth filter and the filter was washed with 1 volume cold STC1700 (1.2 M sorbitol/10 mM Tris.HCl pH 7.5/ 50 mM CaCl₂/ 35 mM NaCl). The protoplasts were spun down at 2500 rpm for 10 minutes at 4 °C. The pellet was resuspended in STC1700 and centrifuged again. After resuspending the pellet in STC1700, the protoplasts were counted. STC1700 was added to a final concentration of 2 x 10⁸ protoplasts per ml.

Vector DNA (pAB4-1 or pBLUE-pyrE, 1-10 µg) was pipetted into the bottom of a sterile tube and 1 µl 1M ATA (aurintricarbonic acid) and 100 µl protoplasts (ca. 2 x 10⁷) were added to the DNA. A minus DNA negative control was included in the experiment. After mixing, the protoplasts were incubated at room temperature for 25 minutes. PEG6000 (60% PEG/50 mM CaCl₂/ 10 mM Tris pH 7.5) was added portionwise as follows: 250 µl, mix, 250 µl, mix, 850 µl and mix. The solution was kept at room temperature for 20 minutes. The tubes were then filled with 8 ml STC1700, mixed and centrifuged at 2500 rpm for 10 minutes at 4 C, and the pellet was suspended in 250 µl STC1700. Aloquots of the sample were used for plating on selective medium. For *pyr*⁺ selection, plates were prepared containing 1.5% Daishin agar, 1.2 M sorbitol, 1x AspA with nitrate, 2mM MgSO₄.7 H₂O, 1x trace elements, 0.1% casaminoacids and 1% glucose. If selected for *amdS* (and *pyr*⁺), the plates contained 1.5% Oxoid agar, 1.2 M sorbitol, 2mM MgSO₄.7 H₂O, 1x trace elements, 1% glucose, 1x AspA without nitrate, 15mM CsCl and 10mM acetamide or acrylamide. The plates were incubated at 30 or 35 °C.

The spores and viable protoplasts before and after PEG6000 treatment were counted by plating dilutions in STC1700 on minimal medium plates with nitrate and with or without sorbitol. 100 µl of 10⁻¹, 10⁻² and 10⁻³ dilutions were plated on plates without sorbitol to count for spores and 100 µl of 10⁻², 10⁻³, 10⁻⁴ and 10⁻⁵ dilutions were plated on plates with sorbitol to count the viable protoplasts.

Results of the transformations are shown in Table I.

### H. PROTEIN/BIOMASS RATIOS

For *Chrysosporium, Trichoderma,* and *Aspergillus* strains producing cellulases or amylases, total dry solids were determined by passing a measured aliquot of the whole broth through a preweighed filter, washing with deionized water, and drying the cake and filter overnight at 60 °C and for one hour at 100 °C. After cooling in a dessicator, biomass was determined by subtracting the weight of the filter from the weight of the dry filter plus filter cake and dividing by the volume of broth removed.

For *Trichoderma* and *Aspergillus* strains, the biomass was assumed to be equal to the total dry solids as there was little insoluble material other than biomass at the time measurements were taken. For *Chrysosporium* strains producing cellulase, there was a significant quantity of cellulose in the medium, so biomass was determined as the difference between total dry solids and cellulose. Cellulose was assayed as follows.

Measured aliquots of whole broth were centrifuged to remove solids and the supernatant was discarded. The pellet was resuspended into a volume of 0.1 N NaOH equal to the original broth volume and one tenth volume of 0.5 N NaOH was added. The mixture was incubated for four hours at 65 °C. This treatment dissolved everything except the cellulose. The alkaline mixture was cooled and centrifuged, and the supernatant was discarded. The resulting pellet was washed twice by resuspension in deionized water and centrifugation. The washed pellet was resuspended in deionized water, transferred to a preweighed pan and dried as described above. Cellulose concentration was determined dividing the dry weight by the volume of the aliquot assayed.

Protein was determined by the Bradford dye-binding procedure (M. Bradford, 1976, Anal. Biochem. 72:248) using an immunoglobulin standard. Protein/biomass ratios for selected expressed proteins in various filamentous fungal strains are presented in Table J.

**Table J**

| Enzyme | Strain | g Protein per g Biomass |
|---|---|---|
| Neutral Cellulase | Chrysosporium lucknowense *UV18-25* | 8.2 |
| Neutral Cellulase | *Chrysosporium lucknowense* UV26-2 | 6.0 |
| α-Amylase | Aspergillus oryzae *108-318* | 0.89 |
| Glucoamylase | Aspergillus niger | 0.78 |
| Glucoamylase | Aspergillus niger | 1.11 |
| Acid Cellulase | Trichoderma reesei *A-34* | 0.89 |
| Acid Cellulase | Trichoderma reesei *A-1391* | 0.65 |
| Xylanase | Trichoderma reesei *X-252* | 2.4 |

### I. EXPRESSION AND SECRETION OF GREEN FLUORESCENT PROTEIN IN A. SOJAE AND C. LUCKNOWENSE

As an example of a versatile and easily screenable reporter protein, Green Fluorescent Protein (GFP) from the jellyfish *Aequoria victoria* was expressed in *A. sojae and C. lucknowense*. Vectors carrying GFP (A. Santerre Henriksen et al., Microbiology. 1999, 145:729-734) and Glucoamylase-GFP fusion genes (pGPDGFP, C. Gordon et al., Microbiology. 2000 146:415-26) were modified by replacing the glaA promoter with the constitutively-expressed *A. nidulans* gpdA promoter. The vectors were introduced into *A. sojae* by cotransformation, using either the *pyrG* or *amdS* selection marker. Vector pGPDGFP and its derivatives were introduced in *Chrysosporium* by cotransformation using either the *pyrE* or *amdS* selection marker. Expression resulted in brightly fluorescent *A. sojae* and *Chrysosporium* transformants, confirming expression of GFP by both vectors. Fluorescence of culture supernatants from transformants expressing Glucoamylase-GFP fusion protein indicated secretion of the fluorescently active fusion protein. Expression of fluorescent protein was also observed in spores (or spore-like propagules) obtained from the various transformants expressing the non-secreted cytoplasmic version of the fluorescent proteins.

### J. TRANSFER OF FUNGAL GROWTH UNITS

The wells of a 96-well microtiter plate are loaded with an appropriate medium, either manually with a multi-channel pipet or by means of an automated plate-handling system. A large volume increases the chance of cross-infection, whereas to avoid problems with evaporation the volume should not be too small. If using the COSTAR(TM) 3799 roundbottom plate, for example, 150 µl is an appropriate volume to work with. Plates are inoculated with spores from plate-grown colonies using toothpicks for transfer. Alternatively, plates can be inoculated by pipetting small aliquots of suspensions of spores, protoplasts or hyphal elements. These suspensions may be derived from isolated spore/protoplast solutions or from microplate grown sporulating cultures. Inoculation can also be carried out from microtiter plates with the use of a pin or a 96-pin tool.

Subsequently plates are incubated at 35°C. To minimize evaporation, lidded plates may be employed, or the plates may be sealed with a membrane that allows exchange of O₂, H₂O and CO₂ and sticks to the surface of the plate. To further limit evaporation, a controlled-atmosphere incubator may be used.

After three to four days of incubation, the amount of biomass is appropriate for efficient transfer to new microtiter plates containing fresh medium. For preparation of replica plates, a 96-pin tool is used. Daughter plates having different arrangements of the cultures may be prepared by manual or robotic pipetting or pin transfer. To ensure the presence of transferable reproducing elements on the transfer pins, the pin tool is submerged into the microtiter plate culture and shaken for 20 seconds. The pin tool is then carefully removed from the starting plate and a print is made into a new microtiter plate. A similarly efficient transfer procedure can also be achieved by using a multi-channel pipet, transferring about 1 µl of the parent microtiter plate culture. In both cases efficient transfer is achieved due to the presence of the transferable reproductive elements, such as spores, spore-like propagules, protoplasts, or hyphal or mycelial fragments. Protoplasts may be generated in the microplate wells by treatment with cell wall degrading enzymes and then transfer these protoplasts. Protoplast formation in microplates has been described by C. van Zeijl et al., J Biotechnol. 1997 59:221-224.

A further improvement of the transfer is obtained by incubating the microtiter plate cultures on a microtiter plate shaker at 35 °C. This increases the number of transferable reproductive elements in the cultures. To store the microtiter plate cultures, glycerol is added to a 15 % end concentration, and the plates are stored at -80 °C. For subsequent transfer experiments plates are defrosted and transfer is carried out as described before. Efficient transfer with wild-type or commercial strains of *A. niger* and *A. sojae* was not feasible under the conditions used here, as these strains showed vigorous surface growth and aerial sporulation after one day. Aerial sporulation causes massive cross-contamination during transfer, and surface growth covering the wells subsequently precludes a large proportion of known assay methods.

### K. CONSTRUCTION OF A FUNGAL EXPRESSION LIBRARY FOR GENE DISCOVERY

Based on the fungal expression vector pAN52-1NOT (EMBL accession Z32524) or one of its derivatives, a vector was constructed in which a unique BamHI cloning site is present directly downstream of the constitutively expressed broad fungal host range promoter for the *A. nidulans* gpdA gene (P. Punt et al., J. Biotechnol. 1991 17:19-33). This vector was constructed in such a way that genomic DNA fragments carrying a translation start codon (ATG) may be expressed. To provide a selection marker for this vector, a NotI-BamHI fragment from pBLUEpyrE was cloned in the NotI-BglII digested expression vector termed pAN52-BamHI, resulting in vector pAN52-pyrE. *Chrysosporium* genomic DNA fragments in a size range of 3-6 kb were obtained partial Sau3A digestion. After ligation of these fragments into the BamHI-digested expression vector pAN52-pyrE, a number of recombinant clones sufficient to cover the full *Chrysosporium* genome several times was obtained. A number of these clones were pooled to cover at least 5-10 fungal genome equivalents. Plasmid DNA of these pools was prepared and used for transformation of *Chrysosporium* pyr5 or *Aspergillus* pyrE mutants. Transformant collections were generated in a microplate format as described above, and used for further functional/activity screening. Alternatively, an expression library may be constructed using specifically regulated *Chrysosporium* promoters, as described in PCT/NL99/00618.

References cited in Examples
1. Calmels T.P., Martin F., Durand H., and Tiraby G. (1991) Proteolytic events in the processing of secreted proteins in fungi. J. Biotechnol. 17(1):51-66.
2. Punt P.J., Dingemanse M.A., Jacobs-Meijsing B.J., Pouwels P.H., and van den Hondel C.A. (1988) Isolation and characterization of the glyceraldehyde-3-phosphate dehydrogenase gene ofAspergillus nidulans. Gene 69(1):49-57.
3. Shoemaker S., Schweickart V., Ladner M., Gelfand D., Kwok S., Myambo K., and Innis M. (1983) Molecular cloning of exo-cellobiohydrolase I derived from Trichoderma reesei strain L27. Bio/Technology Oct.:691-696.
4. Drocourt D., Calmels T., Reynes J.P., Baron M., and Tiraby G. (1990) Cassettes of the Streptoalloteichus hindustanus ble gene for transformation of lower and higher eukaryotes to phleomycin resistance. Nucleic Acids Res. 18(13):4009.
5. Mullaney E.J., Hamer J.E., Roberti K.A., Yelton M.M., and Timberlake W.E. (1985) Primary structure of the trpC gene from Aspergillus nidulans. Mol. Gen. Genet. 199(1):37-45.
6. Yanisch-Perron C., Vieira J., and Messing J. (1987) Improved M13 phage cloning vectors and host strains: nucleotide sequences of the M13mp18 and pUC19 vectors. Gene 33:103-119.
7. Durand H., Baron M., Calmels T., and Tiraby G. (1988) Classical and molecular genetics applied to Trichoderma reesei for the selection of improved cellulolytic industrial strains, in Biochemistry and genetics of cellulose degradation, J.P. Aubert, Editor. Academic Press. pp. 135-151.
8. Lowry O.H., Rosebrough N.J., Farr A.L., and Randall R.J. (1951) Protein measurements with the folin phenol reagent. J. Biol. Chem 193, 265-275.
9. Parriche M., Bousson J.C., Baron M., and Tiraby G. Development of heterologous protein secretion systems in filamentous fungi. in 3rd European Conference on Fungal Genetics. 1996. Münster, Germany.
10. Baron M., Tiraby G., Calmels T., Parriche M., and Durand H. (1992) Efficient secretion of human lysozyme fused to the Sh-ble phleomycin resistance protein by the fungus Tolypocladium geodes. J. Biotechnol. 24(3):253-266.
11. Jeenes D.J., Marczinke B., MacKenzie D.A., and Archer D.B. (1993) A truncated glucoamylase gene fusion for heterologous protein secretion from Aspergillus niger. FEMS Microbiol. Lett. 107(2-3):267-271.
12. Stone P.J., Makoff A.J., Parish J.H., and Radford A. (1993) Cloning and sequence-analysis of the glucoamylase gene of neurospora-crassa. Current Genetics 24(3):205-211.
13. Mörsky P. (1983) Turbidimetric determination of lysozyme with Micrococcus lysodeikticus cells: Reexamination of reaction conditions. Analytical Biochem. 128:77-85.
14. Paluh J.L., Orbach M.J., Legerton T.L., and Yanofsky C. (1988) The cross pathway control gene of Neurospora crassa, cpc-1, encodes a protein similar to GCN4 of yeast and the DNA-binding domain of the oncogene v-jun-encoded protein. Proc. Natl. Acad. Sci. USA 85(11):3728-32.
15. Nakari T., Onnela M.L., Ilmen M., Nevalainen K., and Penttilä M. (1994) Fungal promoters active in the presence of glucose, International patent application WO 94/04673
16. Torronen A., Mach R.L., Messner R., Gonzalez R., Kalkkinen N., Harkki A., and Kubicek C.P. (1992) The two major xylanases from Trichoderma reesei: characterization of broth enzymes and genes. Biotechnology 10(11):1461-5.
17. Farkas V. (1985) Novel media for detection of microbial producers of cellulase and xylanase. FEMS Microbiol. Letters 28:137-140.
18. Miller G.L. (1959) Use of dinitrosalicylic acid reagent for determination of reducing sugar. Anal. Chem. 31:426-428.
19. Punt P.J., Mattern I.E., van den Hondel C.A.M.J.J. (1988) A vector for Aspergillus transformation conferring phleomycin resistance. Fungal Genetics Newsletter 35, 25-30.

## Claims

1. A method of expressing a plurality of proteins encoded by a library of DNA vectors, wherein the library of vectors comprises a plurality of different vectors, each different vector comprising a different protein-encoding nucleic acid sequence, said nucleic acid sequence being operably linked to an expression-regulating region and optionally a secretion signal encoding sequence, the method comprising the steps of:
(a) providing a plurality of individual filamentous fungi, said fungi having a phenotype **characterized by** growth in suspension and **characterized by** the production of transferable reproductive elements which are monoclonal and readily separated from one another in suspension;
(b) stably transforming said filamentous fungi with said library of DNA vectors so as to introduce into each of a plurality of the individual fungi at least one homologous or heterologous protein-encoding nucleic acid sequence;
(c) culturing the transformed filamentous fungi in suspension under conditions conducive to formation of transferable reproductive elements;
(d) separating from one another a plurality of transferable reproductive elements; and
(e) culturing into monoclonal cultures or monoclonal colonies the individual transferable reproductive elements, under conditions conducive to expression of the proteins encoded by the homologous or heterologous protein-encoding nucleic acid sequences.

2. A method of screening a plurality of proteins encoded by a library of DNA vectors for an activity or property of interest, comprising the steps of:
(a) expressing the plurality of proteins in monoclonal filamentous fungal cultures or monoclonal filamentous fungal colonies, by the method of claim 1; and
(b) screening individual clonal cultures or clonal colonies for the activity or property of interest.

3. A method of producing a DNA molecule encoding a protein having an activity or property of interest, comprising the steps of:
(a) expressing a plurality of proteins in monoclonal filamentous fungal cultures or monoclonal filamentous fungal colonies, by the method of claim 1;
(b) screening individual clonal cultures or clonal colonies for the activity or property of interest; and
(c) isolating DNA from a clonal culture or clonal colony exhibiting the activity or property of interest.

4. A method of producing the nucleotide sequence of a DNA molecule encoding a protein having an activity or property of interest, comprising the steps of:
(a) isolating DNA from a clonal culture or clonal colony exhibiting the activity or property of interest, by the method of claim 3; and
(b) sequencing said DNA.

5. A method of producing the amino acid sequence of a protein having an activity or property of interest, comprising the steps of:
(a) producing the DNA sequence of the protein having an activity or property of interest, by the method of claim 4; and
(b) converting said DNA sequence into an amino acid sequence.

6. A method of optimizing a protein's activity or property of interest, comprising the steps of:
(a) providing a library of vectors which comprise DNA sequences encoding mutant forms of the protein;
(b) providing a plurality of individual filamentous fungi, said fungi having a phenotype **characterized by** growth in suspension and by the production of transferable reproductive elements which are monoclonal and readily separated from one another in suspension;
(c) stably transforming said filamentous fungi with said library of DNA vectors so as to introduce into each of a plurality of individual fungi at least one homologous or heterologous protein-encoding nucleic acid sequence;
(d) culturing the transformed filamentous fungi in suspension under conditions conducive to the formation of transferable reproductive elements in suspension;
(e) separating from one another a plurality of transferable reproductive elements;
(f) culturing into clonal cultures or clonal colonies the individual transferable reproductive elements, under conditions conducive to expression of the proteins encoded by the homologous or heterologous protein-encoding nucleic acid sequences;
(g) screening each individual organism, clonal culture, or clonal colony for an expressed protein having the activity or property of interest;
(h) isolating one or more individual organisms, clonal cultures, or clonal colonies that express a protein exhibiting the activity or property of interest;
(i) mutating the DNA from the isolated individual organisms, clonal cultures, or clonal colonies that encodes the protein exhibiting the activity or property of interest;
(j) providing a library of vectors which comprise the mutated DNA sequences obtained in step (i); and
(k) repeating steps (b) through (g), until the property or activity of interest either reaches a desirable level or no longer improves.

7. The method of claim 6, further comprising between steps (h) and (i) the steps of:
culturing one or more of the individual organisms, clonal cultures, or clonal colonies isolated in step (h); isolating the expressed protein exhibiting the activity or property of interest; and
evaluating the isolated protein for the property of interest.

8. The method of any of claims 2-7, wherein the screening step is carried out by high-throughput screening.

9. The method of any one of claims 1-8, wherein the viscosity of the suspension is less than 200 cP.

10. The method of any one of claims 1-8, wherein the viscosity of the suspension is less than 100 cP.

11. The method of any one of claims 1-8, wherein the viscosity of the suspension is less than 60 cP.

12. The method of any one of claims 1-8, wherein the viscosity of the suspension is less than 10 cP.

13. The method of any one of claims 1-8, wherein the vectors comprise a fungal signal sequence.

14. The method of claim 13, wherein the fungal signal sequence is the signal sequence of a fungal gene encoding a protein selected from the group consisting of cellulase, β-galactosidase, xylanase, pectinase, esterase, protease, amylase, polygalacturonase and hydrophobin.

15. The method of any one of claims 1-8, wherein the vectors comprise a nucleotide sequence encoding a selectable marker.

16. The method of any one of claims 1-8, wherein the vectors comprise an expression-regulating region operably linked to the protein-encoding nucleic acid sequence.

17. The method of claim 16, wherein the expression regulating region comprises is an inducible promoter.

18. The method of any one of claims 1-8, wherein the fungus is of the class Euascomycetes.

19. The method of claim 18 wherein the fungus is of the order Onygenales or Eurotiales.

20. The method of any one of claims 1-8, wherein the fungus is of the division Ascomycota, with the proviso that it is not of the order Saccharomycetales.

21. The method of any one of claims 1-8, wherein the fungus is of a genus selected from the group consisting of: Aspergillus, Trichoderma, Chrysosporium, Neurospora, Rhizomucor, Hansenula, Humicola, Mucor, Tolypocladium, Fusarium, Penicillium, Talaromyces, Emericella and Hypocrea.

22. The method of claim 21 wherein the fungus is of a genus selected from the group consisting of Aspergillus, Fusarium, Chrysosporium, and Trichoderma.

23. The method of claim 22, wherein the fungus is Chrysosporium strain UV18-25 having accession number VKM F-3631 D, Trichoderma longibrachiatum strain X-252, Aspergillus sojae strain pclA or Aspergillus niger strain pclA.

24. The method of any of claims 1-8, wherein the expressed homologous or heterologous protein is secreted into the culture medium.

25. The method of any of claims 1-8, wherein the transferable reproductive elements are individual fungal cells.

26. The method of any of claims 1-8, wherein the transferable reproductive elements are spores, hyphal fragments or micropellets.

27. The method of any of claims 1-8, wherein the transferable reproductive elements are protoplasts.

28. The method according to claim 2 further comprising (c) isolating the expressed protein.

29. A method for obtaining a transformed filamentous fungal host expressing a protein having an activity or property of interest, comprising the steps of:
(a) screening a plurality of proteins encoded by a library of DNA vectors for an activity or property of interest, by the method of claim 2; and
(b) isolating the monoclonal culture or monoclonal colony expressing the activity or property of interest.

## Patentansprüche

1. Ein Verfahren zur Expression einer Vielzahl von einer Bibliothek von DNA-Vektoren codierter Proteine, wobei die Bibliothek von Vektoren eine Vielzahl verschiedener Vektoren umfasst, wobei jeder verschiedene Vektor eine andere Protein-codierende Nukleinsäure-Sequenz umfasst, wobei besagte Nukleinsäure-Sequenz an eine Expression regulierende Region und gegebenenfalls eine Sekretionssignal-codierende Sequenz operabel gebunden ist, wobei das Verfahren die Schritte umfasst:
(a) Bereitstellen einer Vielzahl einzelner filamentöser Pilze, wobei besagte Pilze einen Phänotyp besitzen, der durch ein Wachstum in Suspension gekennzeichnet ist und durch die Produktion transferierbarer reproduktiver Elemente gekennzeichnet ist, die monoklonal sind und ohne weiteres in Suspension voneinander getrennt sind;
(b) stabiles Transformieren besagter filamentöser Pilze mit besagter Bibliothek von DNA-Vektoren, um in jeden einer Vielzahl der einzelnen Pilze wenigstens eine homologe oder heterologe Protein-codierende Nukleinsäure-Sequenz einzuführen;
(c) Kultivieren der transformierten filamentösen Pilze in Suspension unter Bedingungen, die für eine Bildung transferierbarer reproduktiver Elemente förderlich sind;
(d) Trennen einer Vielzahl transferierbarer reproduktiver Elemente voneinander; und
(e) Kultivieren der einzelnen transferierbaren reproduktiven Elemente in monoklonalen Kulturen oder monoklonalen Kolonien unter Bedingungen, die für eine Expression der von den homologen oder heterologen Protein-codierenden Nukleinsäure-Sequenzen codierten Proteine förderlich sind.

2. Ein Verfahren zum Sichten einer Vielzahl von einer Bibliothek von DNA-Vektoren codierter Proteine auf eine Aktivität oder Eigenschaft von Interesse, umfassend die Schritte:
(a) Exprimieren der Vielzahl an Proteinen in monoklonalen filamentösen Pilzkulturen oder monoklonalen filamentösen Pilzkolonien mit dem Verfahren nach Anspruch 1; und
(b) Sichten einzelner klonaler Kulturen oder klonaler Kolonien auf die Aktivität oder Eigenschaft von Interesse.

3. Ein Verfahren zur Herstellung eines DNA-Moleküls, das ein Protein mit einer Aktivität oder Eigenschaft von Interesse codiert, umfassend die Schritte:
(a) Exprimieren einer Vielzahl an Proteinen in monoklonalen filamentösen Pilzkulturen oder monoklonalen filamentösen Pilzkolonien mit dem Verfahren nach Anspruch 1; und
(b) Sichten einzelner klonaler Kulturen oder klonaler Kolonien auf die Aktivität oder Eigenschaft von Interesse; und
(c) Isolieren von DNA aus einer klonalen Kultur oder klonalen Kolonie, die die Aktivität oder Eigenschaft von Interesse zeigt.

4. Ein Verfahren zur Herstellung der Nukleotid-Sequenz eines DNA-Moleküls, das ein Protein mit einer Aktivität oder Eigenschaft von Interesse codiert, umfassend die Schritte:
(a) Isolieren von DNA aus einer klonalen Kultur oder klonalen Kolonie, die die Aktivität oder Eigenschaft von Interesse zeigt, mit dem Verfahren von Anspruch 3; und
(b) Sequenzieren besagter DNA.

5. Ein Verfahren zur Herstellung der Aminosäure-Sequenz eines Proteins mit einer Aktivität oder Eigenschaft von Interesse, umfassend die Schritte:
(a) Herstellen der DNA-Sequenz des Proteins mit einer Aktivität oder Eigenschaft von Interesse, mit dem Verfahren von Anspruch 4; und
(b) Konvertieren besagter DNA-Sequenz in eine Aminosäure-Sequenz.

6. Ein Verfahren zur Optimierung einer Aktivität oder Eigenschaft von Interesse eines Proteins, umfassend die Schritte:
(a) Bereitstellen einer Bibliothek von Vektoren, die DNA-Sequenzen umfassen, die mutierte Formen des Proteins codieren;
(b) Bereitstellen einer Vielzahl einzelner filamentöser Pilze, wobei besagte Pilze einen Phänotyp besitzen, der durch ein Wachstum in Suspension gekennzeichnet ist und durch die Produktion transferierbarer reproduktiver Elemente gekennzeichnet ist, die monoklonal sind und ohne weiteres in Suspension voneinander getrennt sind;
(c) stabiles Transformieren besagter filamentöser Pilze mit besagter Bibliothek von DNA-Vektoren, um in jeden einer Vielzahl einzelner Pilze wenigstens eine homologe oder heterologe Protein-codierende Nukleinsäure-Sequenz einzuführen;
(d) Kultivieren der transformierten filamentösen Pilze in Suspension unter Bedingungen, die für die Bildung transferierbarer reproduktiver Elemente förderlich sind;
(e) Trennen einer Vielzahl transferierbarer reproduktiver Elemente voneinander; und
(f) Kultivieren der einzelnen transferierbaren reproduktiven Elemente in klonalen Kulturen oder klonalen Kolonien unter Bedingungen, die für eine Expression der von den homologen oder heterologen Protein-codierenden Nukleinsäure-Sequenzen codierten Proteine förderlich sind.
(g) Sichten eines/r jeden einzelnen Organismus, klonalen Kultur oder klonalen Kolonie auf ein exprimiertes Protein mit der Aktivität oder Eigenschaft von Interesse;
(h) Isolieren eines/r oder mehrerer einzelner Organismen, klonaler Kulturen oder klonaler Kolonien, die ein Protein exprimieren, das die Aktivität oder Eigenschaft von Interesse zeigt;
(i) Mutieren der DNA aus den isolierten einzelnen Organismen, klonalen Kulturen oder klonalen Kolonien, die das Protein codiert, das die Aktivität oder Eigenschaft von Interesse zeigt;
(j) Bereitstellen einer Bibliothek von Vektoren, die die in Schritt (i) erhaltenen mutierten DNA-Sequenzen umfassen; und
(k) Wiederholen der Schritte (b) bis (g), bis die Eigenschaft oder Aktivität von Interesse entweder ein erwünschtes Niveau erreicht oder nicht mehr zu verbessern ist.

7. Das Verfahren nach Anspruch 6, weiterhin umfassend zwischen Schritt (h) und (i) die Schritte:
Kultivieren eines/r oder mehrerer der in Schritt (h) isolierten einzelnen Organismen, klonalen Kulturen oder klonalen Kolonien; Isolieren des exprimierten Proteins, das die Aktivität oder Eigenschaft von Interesse zeigt; und Bewerten des isolierten Proteins nach der Eigenschaft von Interesse.

8. Das Verfahren nach einem der Ansprüche 2 bis 7, wobei der Sichtungsschritt mit einer Hochdurchsatz-Sichtung durchgeführt wird.

9. Das Verfahren nach einem der Ansprüche 1 bis 8, wobei die Viskosität der Suspension kleiner als 200 cP ist.

10. Das Verfahren nach einem der Ansprüche 1 bis 8, wobei die Viskosität der Suspension kleiner als 100 cP ist.

11. Das Verfahren nach einem der Ansprüche 1 bis 8, wobei die Viskosität der Suspension kleiner als 60 cP ist.

12. Das Verfahren nach einem der Ansprüche 1 bis 8, wobei die Viskosität der Suspension kleiner als 10 cP ist.

13. Das Verfahren nach einem der Ansprüche 1 bis 8, wobei die Vektoren eine pilzliche Signalsequenz umfassen.

14. Das Verfahren nach Anspruch 13, wobei die pilzliche Signalsequenz die Signalsequenz eines Pilzgens ist, das ein Protein codiert, das aus der Gruppe ausgewählt ist, bestehend aus Cellulase, β-Galactosidase, Xylanase, Pectinase, Esterase, Protease, Amylase, Polygalacturonase und Hydrophobin.

15. Das Verfahren nach einem der Ansprüche 1 bis 8, wobei die Vektoren eine Nukleinsäure-Sequenz umfassen, die einen selektierbaren Marker codiert.

16. Das Verfahren nach einem der Ansprüche 1 bis 8, wobei die Vektoren eine Expression regulierende Region umfassen, die an die Protein-codierende Nukleinsäure-Sequenz operabel gebunden ist.

17. Das Verfahren nach Anspruch 16, wobei die Expression regulierende Region einen induzierbaren Promotor umfasst.

18. Das Verfahren nach einem der Ansprüche 1 bis 8, wobei der Pilz zur Klasse der Euascomyceten gehört.

19. Das Verfahren nach Anspruch 18, wobei der Pilz zur Ordnung der Onygenales oder Eurotiales gehört.

20. Das Verfahren nach einem der Ansprüche 1 bis 8, wobei der Pilz zur Abteilung der Ascomycota gehört, unter der Bedingung, dass er nicht zur Ordnung der Saccharomycetales gehört.

21. Das Verfahren nach einem der Ansprüche 1 bis 8, wobei der Pilz zu einer Gattung gehört, die aus der Gruppe ausgewählt ist, bestehend aus: Aspergillus, Trichoderma, Chrysosprorium, Neurospora, Rhizomucor, Hansenula, Humicola, Mucor, Tolypocladium, Fusarium, Penicillium, Talaromyces, Emericella und Hypocrea.

22. Das Verfahren nach Anspruch 21, wobei der Pilz zu einer Gattung gehört, die aus der Gruppe ausgewählt ist, bestehend aus Aspergillus, Fusarium, Chrysosprium und Trichoderma.

23. Das Verfahren nach Anspruch 22, wobei der Pilz Chrysosporium Stamm UV 18-25 mit der Hinterlegungsnummer VKM F-3631 D, Trichoderma longibrachiatum Stamm X-252, Aspergillus sojae Stamm pclA oder Aspergillus niger Stamm pclA ist.

24. Das Verfahren nach einem der Ansprüche 1 bis 8, wobei das exprimierte homologe oder heterologe Protein in das Kulturmedium sezerniert wird.

25. Das Verfahren nach einem der Ansprüche 1 bis 8, wobei die transferierbaren reproduktiven Elemente einzelne Pilzzellen sind.

26. Das Verfahren nach einem der Ansprüche 1 bis 8, wobei die transferierbaren reproduktiven Elemente Sporen, Hyphenfragmente oder Mikropellets sind.

27. Das Verfahren nach einem der Ansprüche 1 bis 8, wobei die transferierbaren reproduktiven Elemente Protoplasten sind.

28. Das Verfahren gemäß Anspruch 2, außerdem umfassend (c) Isolieren des exprimierten Proteins.

29. Ein Verfahren zur Gewinnung eines transformierten filamentösen pilzlichen Wirts, der ein Protein mit einer Aktivität oder Eigenschaft von Interesse exprimiert, umfassend die Schritte:
(a) Sichten einer Vielzahl von einer Bibliothek von DNA-Vektoren codierter Proteine auf eine Aktivität oder Eigenschaft von Interesse mit dem Verfahren nach Anspruch 2; und
(b) Isolieren der monoklonalen Kultur oder monoklonalen Kolonie, die die Aktivität oder Eigenschaft von Interesse exprimiert.

## Revendications

1. Procédé permettant l'expression d'un ensemble de protéines codées par une bibliothèque de vecteurs d'ADN selon lequel la bibliothèque de vecteurs comporte un ensemble de vecteurs différents, chacun de ces vecteurs différents comprenant une séquence d'acides nucléiques codant pour une protéine différente, cette séquence d'acides nucléiques étant opérationnellement liée à une région de régulation de l'expression et le cas échéant à une séquence codant pour un signal de sécrétion, le procédé comprenant les étapes consistant en :
(a) se procurer une pluralité de champignons filamenteux individuels, ces champignons ayant une phénotype **caractérisée par** la croissance en suspension et **caractérisée par** la production d'éléments reproducteurs transférables monoclonaux et facilement séparés les uns des autres en suspension,
(b) transformer de façon stable les champignons filamenteux avec la bibliothèque de vecteurs d'ADN de façon à introduire dans chaque champignon d'un ensemble de champignons individuels au moins une séquence d'acides nucléiques codant pour une protéine homologue ou hétérologue,
(c) cultiver les champignons filamenteux transformés en suspension sous des conditions favorables à la formation d'éléments reproducteurs transférables,
(d) séparer les uns des autres un ensemble d'éléments reproducteurs transférables, et
(e) cultiver les éléments reproducteurs transférables dans des cultures monoclonales ou des colonies monoclonales sous des conditions favorables à l'expression des protéines codées par les séquences d'acides nucléiques codant pour les protéines homologues ou hétérologues.

2. Procédé de criblage d'un ensemble de protéines codées par une bibliothèque de vecteurs d'ADN pour une activité ou une propriété intéressante, comprenant les étapes consistant à :
(a) exprimer l'ensemble des protéines dans des cultures de champignons filamenteux monoclonaux ou des colonies de champignons filamenteux monoclonaux selon le procédé conforme à la revendication 1, et
(b) cribler les cultures clonales individuelles ou les colonies clonales individuelles pour l'activité ou la propriété intéressante.

3. Procédé de production d'une molécule d'ADN codant pour une protéine ayant une activité ou une propriété intéressante, comprenant les étapes consistant à :
(a) exprimer un ensemble de protéines dans des cultures de champignons filamenteux monoclonales ou des colonies de champignons filamenteux monoclonales selon le procédé conforme à la revendication 1,
(b) cribler les cultures clonales ou les colonies clonales individuelles pour l'activité ou la propriété intéressante, et
(c) isoler l'ADN d'une culture clonale ou d'une colonie clonale présentant l'activité ou la propriété intéressante.

4. Procédé de production de la séquence nucléotidique d'une molécule d'ADN codant pour une protéine ayant une activité ou une propriété intéressante, comprenant les étapes consistant à :
(a) isoler l'ADN d'une culture clonale ou d'une colonie clonale présentant l'activité ou la propriété intéressante selon le procédé conforme à la revendication 3, et
(b) effectuer le séquençage de cet ADN.

5. Procédé de production d'une séquence d'acides aminés d'une protéine ayant une activité ou une propriété intéressante comprenant les étapes consistant à :
(a) produire la séquence d'ADN d'une protéine ayant une activité ou une propriété intéressante selon le procédé conforme à la revendication 4, et
(b) transformer la séquence d'ADN en une séquence d'acides aminés.

6. Procédé d'optimisation d'une activité ou d'une propriété intéressante d'une protéine comprenant les étapes consistant à :
(a) se procurer une bibliothèque de vecteurs qui comprennent des séquences d'ADN codant pour des formes mutantes de la protéine,
(b) se procurer un ensemble de champignons filamenteux individuels, ces champignons ayant un phénotype **caractérisé par** une croissance en suspension et par la production d'éléments reproducteurs transférables monoclonaux et facilement séparables les uns des autres en suspension,
(c) transformer de façon stable ces champignons filamenteux avec la bibliothèque de vecteurs d'ADN de façon à introduire dans chaque champignon d'un ensemble de champignons individuels au moins une séquence d'acides nucléiques codant pour une protéine homologue ou hétérologue,
(d) cultiver les champignons filamenteux transformés en suspension sous des conditions conduisant à la formation d'éléments reproducteurs transférables,
(e) séparer les uns des autres un ensemble d'éléments reproducteurs transférables,
(f) cultiver les éléments reproducteurs transférables dans des cultures clonales ou des colonies clonales sous des conditions favorables à l'expression des protéines codées par les séquences d'acides nucléiques codant pour la protéine homologue ou hétérologue,
(g) cribler chaque organisme individuel, culture clonale ou colonie clonale pour une protéine exprimée ayant l'activité ou la propriété intéressante,
(h) isoler au moins un organisme individuel, une culture clonale ou une colonie clonale qui exprime une protéine présentant l'activité ou la propriété intéressante,
(i) effectuer la mutation de l'ADN des organismes individuels, cultures clonales ou colonies clonales isolés qui code pour la protéine présentant l'activité ou la propriété intéressante,
(j) se procurer une bibliothèque de vecteurs qui comprennent les séquences d'ADN ayant subi une mutation obtenue dans l'étape (i), et
(k) répéter les étapes (b) à (g) jusqu'à ce que la propriété ou l'activité intéressante atteigne un niveau désirable ou n'augmente plus.

7. Procédé selon la revendication 6 comprenant en outre entre les étapes (h) et (i) les étapes consistant à cultiver au moins l'organisme individuel, la culture clonale ou la colonie clonale isolée dans l'étape (h), isoler la protéine exprimée présentant l'activité ou la propriété intéressante et évaluer la protéine isolée pour ce qui est de la propriété intéressante.

8. Procédé selon l'une des revendications 2-7 selon lequel l'étape de criblage est mise en oeuvre par criblage à débit élevé.

9. Procédé selon l'une des revendications 1-8 selon lequel la viscosité de la suspension est inférieure à 200 cP.

10. Procédé selon l'une des revendications 1-8 selon lequel la viscosité de la suspension est inférieure à 100 cP.

11. Procédé selon l'une des revendications 1-8 selon lequel la viscosité de la suspension est inférieure à 60 cP.

12. Procédé selon l'une des revendications 1-8 selon lequel la viscosité de la suspension est inférieure à 10 cP.

13. Procédé selon l'une des revendications 1-8 selon lequel le vecteur comprend un peptide de signal fongique.

14. Procédé selon la revendication 13 selon lequel le peptide signal fongique est le peptide signal d'un gène fongique codant pour une protéine choisie dans le groupe formé par la cellulase, la β-galactosidase, la xylanase, la pectinase, l'estérase, la protéase, l'amylase, la polygalacturonase et l'hydrophobine.

15. Procédé selon l'une des revendications 1-8 selon lequel les vecteurs comportent une séquence nucléotidique codant pour un marqueur sélectionnable.

16. Procédé selon l'une des revendications 1-8 selon lequel les vecteurs comportent une région de régulation de l'expression opérationnellement liée à la séquence d'acides nucléiques codant pour la protéine.

17. Procédé selon la revendication 16 selon lequel la région de régulation de l'expression comprend un promoteur inducteur.

18. Procédé selon l'une des revendications 1-8 selon lequel le champignon est un champignon appartenant à la classe des Euascomycetes.

19. Procédé selon la revendication 18 selon lequel le champignon appartient à l'ordre des Onygénales ou des Eurotiales.

20. Procédé selon l'une des revendications 1-8 selon lequel le champignon appartient à la division Ascomycota étant précisé qu'il n'appartient pas à l'ordre des Saccharomycetales.

21. Procédé selon l'une des revendications 1-8 selon lequel le champignon est un champignon dont le genre est choisi dans le groupe formé par les genres Aspergillus, Trichoderma, Chrysosporium, Neurospora, Rhizomucor, Hansenula, Humicola, Mucor, Tolypocladium, Fusarium, Penicillium, Talaromyces, Emericella et Hypocrea.

22. Procédé selon la revendication 21 selon lequel le champignon est un champignon dont le genre est choisi dans le groupe formé par les genres Aspergillus, Fusarium, Chrysosporium et Trichoderma.

23. Procédé selon la revendication 22 selon lequel le champignon est le champignon Chrysosporium souche UV18-25 ayant le numéro de catalogue KM F-3631 D, le champignon Trichoderma longibrachiatum souche X-252, le champignon Aspergillus sojae souche pc1A ou le champignon Aspergillus niger souche pc1A.

24. Procédé selon l'une des revendications 1-8 selon lequel la protéine homologue ou hétérologue exprimée est sécrétée dans le milieu de culture.

25. Procédé selon l'une des revendications 1-8 selon lequel les éléments reproducteurs transférables sont des cellules fongiques individuelles.

26. Procédé selon l'une des revendications 1-8 selon lequel les éléments reproducteurs transférables sont des spores, des fragments d'hyphes ou des micropellets.

27. Procédé selon l'une des revendications 1-8 selon lequel les éléments reproducteurs transférables sont des protoplastes.

28. Procédé selon la revendication 2 comprenant en outre l'étape (c) consistant à isoler la protéine exprimée.

29. Procédé d'obtention d'un hôte fongique filamenteux transformé exprimant une protéine ayant une activité ou une propriété intéressante comprenant les étapes consistant à :
(a) cribler un ensemble de protéines codées par une bibliothèque de vecteurs d'ADN pour une activité ou une propriété intéressante par le procédé conforme à la revendication 2, et
(b) isoler la culture monoclonale ou la colonie monoclonale exprimant l'activité ou la propriété intéressante.
